# EUROPEAN PATENT APPLICATION

(11) **EP 4 360 601 A1**
(43) Date of publication of application: **01.05.2024**
(21) Application number: 22864164.3
(22) Date of filing: 03.08.2022
(51) Int. Cl.: A61F 13/15, A61F 13/49, A61F 13/496, A61F 13/51

(54) **UNDERPANTS-TYPE ABSORBENT ARTICLE AND METHOD FOR MANUFACTURING UNDERPANTS-TYPE ABSORBENT ARTICLE**

(30) Priority: 31.08.2021 JP 2021141414
(71) Applicant: Unicharm Corporation, Ehime 799-0111 (JP)
(72) Inventor: OHTSUBO, Toshifumi, Kanonji-shi, Kagawa 769-1602 (JP); MUKAI, Hirotomo, Kanonji-shi, Kagawa 769-1602 (JP); NAGAI, Takahito, Kanonji-shi, Kagawa 769-1602 (JP); AKINO, Chieri, Kanonji-shi, Kagawa 769-1602 (JP)
(74) Representative: Dolleymores
(86) International application number: PCT/JP2022/029794
(87) International publication number: WO 2023/032578

(57) **Abstract**

The present invention provides an underpants-type absorbent article (1) in which a ventral waist part (20) and a dorsal waist part (30) are joined by side joining zones (40), wherein: the waist parts have a plurality of weld pairs (50S) that trap therebetween and restrict the location of an elastic member (23, 33) in a constricted state in the left-right direction; the plurality of weld pairs (50S) include a first weld pair (50S1) adjacent to the inner side of the side joining zones (40) in the left-right direction and a second weld pair (50S2) adjacent to the inner side of the first weld pair (50S1) in the left-right direction; and, when the underpants-type absorbent article (1) in an elongated state is viewed in the front-back direction, the centerline of the part of the elastic member (23, 33) located in the side joining zones (40) is inclined relative to the centerline of the part of the elastic member (23, 33) located between the first weld pair (50S1) and the second weld pair (50S2).

## Description

### FIELD

The present invention relates to an underpants-shaped absorbent article and a method for manufacturing an underpants-shaped absorbent article.

### BACKGROUND

Patent Document 1 discloses an underpants-shaped absorbent article in which the two side portions of a front portion located on the wearer's front side and the two side portions of a back portion located on the wearer's back side are joined by a pair of the side seal portion. The constituent sheets of the front portion and the constituent sheets of the back portion are each intermittently joined by multiple fusion bonded regions, and the string-like elastic members placed between sheets extend between multiple fusion bonded regions lined up along the lengthwise direction. In addition, while the elastic members are fixed in a pair of outer fixing regions arranged in the two lateral ends of the front portion and the two lateral ends of the back portion, the elastic members are not fixed to any sheet, between the pair of outer fixing regions. Therefore, when the elastic member contracts, the constituent sheets of the front portion and the constituent sheets of the back portion expand between adjacent fusion bonded regions, and hollow portions are formed between sheets.

### [CITATION LIST]

### [PATENT LITERATURE]

[Patent Document 1] WO2019/123549

### SUMMARY

### [TECHNICAL PROBLEM]

The elastic members are generally fixed with an adhesive to constituent sheets of the front portion or the back portion. However, by not fixing the elastic members with the adhesive between the outer fixing regions of the front portion and back portion as in Patent Document 1, it is possible to maintain the flexibility of the front portion and the back portion (waist portion). However, by simply fixing the two ends of the elastic members with the adhesive as in Patent Document 1, there is a risk that the elastic members fall out of the outer fixing regions due to the contractive force of the elastic members. Further, in Patent Document 1, the side seal portions and the fusion bonded regions are formed with a focus on the ease with which the side seal portions can be torn apart, and therefore there is a risk that the elastic members are severed when forming the side seal portion. In this case, the stretchability in the ront portion and the back portion (the waist portion) decreases.

The present invention was achieved in light of conventional problems such as that described above, an aspect of the present invention is to provide an underpants-shaped absorbent article in which the flexibility of its waist portion is maintained while also maintaining the stretchability of the waist portion by suppressing the falling off and/or severing of elastic members provided in the waist portion.

### [SOLUTION TO PROBLEM]

A main aspect of the present invention of achieving the above-described aspect is an underpants-shaped absorbent article having a vertical direction, a lateral direction and a front-back direction that intersect each other, the underpants-shaped absorbent article including: an absorbent main body; and a waist portion in which a front waist portion and a back waist portion are joined by a pair of side-joining-portion regions in two ends in the lateral direction, in the waist portion, a plurality of elastic members being arranged side by side in the vertical direction between a skin-side sheet and a non-skin-side sheet that are joined by a plurality of welded portions, the plurality of elastic members stretching and contracting in the lateral direction, the plurality of welded portions being located on both sides of the elastic member in the vertical direction, a plurality of welded portion pairs being provided in which the elastic member in a state of contracting in the lateral direction is sandwiched and that restricts a position of the elastic member in the lateral direction with respect to the skin-side sheet and the non-skin-side sheet, the plurality of welded portion pairs having a first welded portion pair and a second welded portion pair, the first welded portion pair being located adjacent to and laterally inside the side-joining-portion region that is located on a one side in the lateral direction, the first welded portion pair sandwiching the first elastic member among the plurality of elastic members, the second welded portion pair being located adjacent to and laterally inside the first welded portion pair, the second welded portion pair sandwiching the first elastic member, when the underpants-shaped absorbent article that is in a stretched state is viewed in the front-back direction, a center line of a portion of the first elastic member that is located in the side-joining-portion region located on a one side in the lateral direction being inclined with respect to a center line of a portion of the first elastic member that is located between the first welded portion pair and the second welded portion pair.

Features of the present invention other than the above will become clear by reading the description of the present specification with reference to the accompanying drawings.

### [ADVANTAGEOUS EFFECT OF THE INVENTION]

According to the present invention, it is possible to provide an underpants-shaped absorbent article in which the flexibility of its waist portion is maintained while also maintaining the stretchability of the waist portion by suppressing the falling off and/or severing of elastic members provided in the waist portion.

### BRIEF DESCRIPTION OF DRAWINGS

FIG. 1 is a schematic perspective view of a diaper 1.
FIG. 2 is a schematic plan view of the diaper 1 in an unfolded and stretched state when viewed from a wearer's skin side.
FIG. 3 is a schematic cross-sectional view taken along line A-A in FIG. 2.
FIG. 4A is a diagram illustrating side-joining-portion regions 40 and welded portion rows 50L provided in a waist portion 2, and FIG. 4B is a schematic enlarged view of a section B in FIG. 4A.
FIGS. 5A and 5B are diagrams illustrating a function of attaching a waist elastic member 23 (33).
FIGS. 6A and 6B are diagrams showing a state in which an end portion of the waist elastic member 23 (33) is in contact with a side joining portion 41.
FIGS. 7A to 7C are diagrams showing a state in which the end portion of the waist elastic member 23 (33) is sandwiched on the side joining portion 41.
FIGS. 8A and 8B are diagrams showing modified examples of the side-joining-portion region 40.
FIG. 9 is a diagram illustrating a length L1 of the side joining portion 41 in the vertical direction.
FIGS. 10A and 10B are diagrams illustrating modified examples of the welded portions 50.
FIG. 11 is an enlarged sectional view of the waist elastic member 23 (33).
FIG. 12 is a diagram illustrating the flow of manufacturing the diaper 1 in a manufacturing line.
FIG. 13A is a diagram illustrating the formation positions of the side joining portions 41, and FIG. 13B is a schematic side view of an ultrasonic welding device 60 as seen in the CD direction.

### DESCRIPTION OF EMBODIMENTS

At least the following matters will be clear with the description of this specification and the attached drawing.

An underpants-shaped absorbent article having a vertical direction, a lateral direction and a front-back direction that intersect each other, the underpants-shaped absorbent article including: an absorbent main body; and a waist portion in which a front waist portion and a back waist portion are joined by a pair of side-joining-portion regions in two ends in the lateral direction, in the waist portion, a plurality of elastic members being arranged side by side in the vertical direction between a skin-side sheet and a non-skin-side sheet that are joined by a plurality of welded portions, the plurality of elastic members stretching and contracting in the lateral direction, the plurality of welded portions being located on both sides of the elastic member in the vertical direction, a plurality of welded portion pairs being provided in which the elastic member in a state of contracting in the lateral direction is sandwiched and that restricts a position of the elastic member in the lateral direction with respect to the skin-side sheet and the non-skin-side sheet, the plurality of welded portion pairs having a first welded portion pair and a second welded portion pair, the first welded portion pair being located adjacent to and laterally inside the side-joining-portion region that is located on a one side in the lateral direction, the first welded portion pair sandwiching the first elastic member among the plurality of elastic members, the second welded portion pair being located adjacent to and laterally inside the first welded portion pair, the second welded portion pair sandwiching the first elastic member, when the underpants-shaped absorbent article that is in a stretched state is viewed in the front-back direction, a center line of a portion of the first elastic member that is located in the side-joining-portion region located on a one side in the lateral direction being inclined with respect to a center line of a portion of the first elastic member that is located between the first welded portion pair and the second welded portion pair.

According to such an underpants-shaped absorbent article, the position (movement) of the elastic member is restricted by the welded portion pair, and therefore, it is possible not to use the adhesive for attaching the elastic member to the waist portion, or to reduce the amount of use of the adhesive, thereby maintaining the flexibility of the waist portion. In addition, the elastic member located in the side-joining-portion region is inclined with respect to the direction of a force that causes the elastic member to come off, and this makes the elastic member less likely to come off fromthe side-joining-portion region. Therefore, the stretchability of the waist portion is maintained.

In such an underpants-shaped absorbent article, the first elastic member extends laterally outside the side-joining-portion region that is located on a one side in the lateral direction.

According to such a sunderpants-shaped absorbent article, the elastic member is located extending over the entire region of the side-joining-portion region in the lateral direction, where the frictional force of the elastic member with the sheet is high. This makes the elastic member less likely to come off from the side-joining-portion region.

In such an underpants-shaped absorbent article, in the side-joining-portion region, a plurality of side joining portions are arranged side by side in the vertical direction, and a part of the first elastic member is in contact with the side joining portion in the side-joining-portion region on a one side in the lateral direction.

According to such an underpants-shaped absorbent article, the center line of the elastic member that is located in the side-joining-portion region is likely to be inclined with respect to the center line of the elastic member that is located between the first and the second welded portion pairs. Additionally, the frictional force is generated between the elastic member and the side joining portion, and this makes the elastic member less likely to come off from the side-joining-portion region.

In such an underpants-shaped absorbent article, in the side-joining-portion region, a plurality of side joining portions are arranged side by side in the vertical direction, and in the side-joining-portion region on a one side in the lateral direction, a part of the first elastic member is sandwiched between the skin-side sheet and the non-skin-side sheet by the side joining portion.

According to such an underpants-shaped absorbent article, the center line of the elastic member that is located in the side-joining-portion region is likely to be inclined with respect to the center line of the elastic member that is located between the first and the second welded portion pairs. Also, a part of the elastic member in the side joining portion is firmly sandwiched between the sheets, and this makes the elastic member less likely to come off from the side-joining-portion region.

In such an underpants-shaped absorbent article, in the side-joining-portion region on a one side in the lateral direction, a part of the second elastic member that is among the plurality of the elastic member are in contact with the side joining portion.

According to such an underpants-shaped absorbent article, the plurality of elastic members included in the waist portion are less likely to come off from the side-joining-portion region, and the stretchability of the waist portion is more likely to be maintained.

In such an underpants-shaped absorbent article, in the side-joining-portion region of another side in the lateral direction, a part of the first elastic member is in contact with the side joining portion.

According to such an underpants-shaped absorbent article, the elastic members are less likely to come off from the side-joining-portion region on both sides in the lateral direction.

In such an underpants-shaped absorbent article, in the side-joining-portion region, a plurality of side joining portions are arranged side by side in the vertical direction, and a gap in the vertical direction between the side joining portions that are located beside the first welded portion pair in the lateral direction is shorter than a gap of the first welded portion pair in the vertical direction.

According to such an underpants-shaped absorbent article, a plurality of the side joining portions are likely to be located in a position that overlaps with respect to the vertical direction a portion between the first welded portion pair. Therefore, a part of the elastic member is more likely to come into contact with the side joining portion or to overlap the side joining portion in the thickness direction. Therefore, the center line of the elastic member that is located in the side-joining-portion region is likely to be inclined with respect to the center line that is located between the first and the second welded portion pairs.

In such an underpants-shaped absorbent article, in the side-joining-portion region, a plurality of side joining portions are arranged side by side in the vertical direction, and a gap in the vertical direction between the side joining portions that are located beside the first welded portion pair in the lateral direction is equal to or greater than a gap of the first welded portion pair in the vertical direction.

According to such an underpants-shaped absorbent article, it is possible to reduce the number of the side joining portions, and the flexibility of the waist portion (side-joining-portion region) is more likely to be maintained.

In such an underpants-shaped absorbent article, in the side-joining-portion region, a plurality of side-joining-portion rows are lined up side-by-side in the lateral direction, in each of the side-joining-portion rows, a plurality of side joining portions being lined up side-by-side in the vertical direction, and the plurality of side-joining-portion rows are spaced apart in the lateral direction.

According to such an underpants-shaped absorbent article, the lateral length of the side-joining-portion region increases, and this makes it possible to increase the length of a portion of the elastic member on which the frictional force with the sheet strongly acts. This makes it possible to suppress the coming off of the elastic member from the side-joining-portion region. Also, the area ratio of the side joining portions in the side-joining-portion region becomes smaller, and this makes it possible to maintain the flexibility of the waist portion (side-joining-portion region).

In such an underpants-shaped absorbent article, in the side-joining-portion region, a plurality of side-joining-portion rows are lined up side-by-side in the lateral direction, in each of the side-joining-portion rows, a plurality of side joining portions being lined up side-by-side in the vertical direction, the side joining portion included in each of the two side-joining-portion rows that are adjacent in the lateral direction is shifted in the vertical direction, and a length in the vertical direction between the side joining portion that is located adjacent to the first welded portion pair in the lateral direction and the side joining portion that is adjacent the foregoing side joining portion and that is shifted with respect to the foregoing side joining portion in the vertical direction is smaller than a diameter of the first elastic member in a state of contracting in the lateral direction.

According to such an underpants-shaped absorbent article, the elastic member is made likely to come into contact with at least one of the side joining portions which are shifted with respect to the vertical direction and which are adjacent in the lateral direction. This makes the elastic member likely to be inclined along the side joining portion, and it is possible to further suppress the coming off of the elastic member from the side-joining-portion region.

In such an underpants-shaped absorbent article, in the side-joining-portion region, a plurality of side joining portions are arranged side by side in the vertical direction, and the side-joining-portion region has a portion where the welded portion and the side joining portion overlap in the thickness direction of the waist portion.

According to such an underpants-shaped absorbent article, the side joining portion becomes likely to be located so as to overlap the elastic member which passes between the welded portion pair. Therefore, a part of the elastic member is more likely to come into contact with the side joining portion or to overlap the side joining portion in the thickness direction. Therefore, the center line of the elastic member that is located in the side-joining-portion region is likely to be inclined with respect to the center line that is located between the first and the second welded portion pairs.

In such an underpants-shaped absorbent article, in at least either one region of the pair of side-joining-portion regions, at least one of the elastic members included in the front waist portion is placed shifted in the vertical direction with respect to the elastic member included in the back waist portion.

According to such an underpants-shaped absorbent article, it is possible to prevent both of the elastic members on the front side and the elastic members on the back side from being severed by the protrusion pattern that forms the side joining portions. In addition, it is possible to reduce the number of materials that overlap in the thickness direction at the locations where the side joining portions are formed, making it possible to firmly join the materials (sheets) at the side joining portions.

In such an underpants-shaped absorbent article, in the side-joining-portion region, a plurality of side joining portions are arranged side by side in the vertical direction, and letting a times be the stretch factor of the first elastic member when the underpants-shaped absorbent article is stretched to a maximum extent in the lateral direction, letting D mm be a width of the first elastic member in the vertical direction when the underpants-shaped absorbent article is stretched to a maximum extent in the lateral direction, letting b times a maximum stretch factor of the first elastic member alone, a vertical length of the side joining portion that is located beside the first welded portion pair in the lateral direction is equal to or less than D × *b*/*a* mm.

According to such an underpants-shaped absorbent article, the vertical length of the side joining portion is likely to be equal to or less than the width in which the elastic member extends without cutting (D × *b*/*a* (mm)), and this makes it possible to prevent the elastic member from being severed by the protrusion pattern that forms the side joining portions.

In such an underpants-shaped absorbent article, a distance in the vertical direction between the two welded portions that constitute the welded portion pair is wider in at least an end portion located on a one side in the lateral direction than in a central portion of the welded portion pair in the lateral direction.

According to such an underpants-shaped absorbent article, the elastic member is likely to move in the vertical direction at the lateral end portion of the welded portion, and the elastic member is likely to be inclined slightly with respect to the lateral direction along the end portion of the welded portion. Therefore, it is possible to prevent the elastic member from coming off from the welded portion pair.

In such an underpants-shaped absorbent article, an oil agent is attached to at least a portion of a surface of the elastic member.

According to such an underpants-shaped absorbent article, even if the elastic member is stepped on by the protrusion pattern, which forms the side joining portions, the elastic member is likely to be pushed out of the protrusion pattern because it is slippery due to the oil agent. Therefore, the elastic member is less likely to be cut off, and it is possible to suppress the coming off of the elastic member from the side-joining-portion region.

In such an underpants-shaped absorbent article, the elastic member is a string-like elastic member in which a plurality of elastic fibers are aggregated.

According to such an underpants-shaped absorbent article, the elastic member is less likely to be severed by the protrusion pattern, which forms the side joining portions, and it is possible to prevent the coming off of the elastic member from the side-joining-portion region.

A method for manufacturing an underpants-shaped absorbent article, the underpants-shaped absorbent article having a vertical direction, a lateral direction and a front-back direction that intersect each other, the underpants-shaped absorbent article including an absorbent main body and a waist portion in which a front waist portion and a back waist portion are joined by a pair of side-joining-portion regions in two ends in the lateral direction, the method including: a step of forming a continuous body of the front waist portion and a continuous body of the back waist portion, by forming a plurality of welded portion pairs when forming a plurality of welded portions after placing a plurality of continuous bodies of an elastic member between a skin-side-sheet continuous body and a non-skin-side-sheet continuous body, the plurality of continuous bodies of the elastic member being in a state of stretching in a direction of transport corresponding to the lateral direction, the plurality of continuous bodies of the elastic member being placed side-by-side in an intersecting direction of the direction of transport, the plurality of welded portions being portions that join the skin-side-sheet continuous body and the non-skin-side-sheet continuous body, the skin-side-sheet continuous body and the non-skin-side-sheet continuous body being continuous in the direction of transport, the plurality of welded portion pairs holding composed of the welded portions that are placed on both sides of each of the plurality of continuous bodies of the elastic member in the intersecting direction, when the elastic member in the underpants-shaped absorbent article contracts in the lateral direction, the plurality of welded portion pairs holding the elastic member and restricting a position of the elastic member in the lateral direction with respect to the skin-side sheet and the non-skin-side sheet; and a step of forming a side-joining-portion region by forming a plurality of side joining portions arranged side-by-side in the intersecting direction, the plurality of side joining portions being portions for joining the continuous body of the front waist portion and the continuous body of the back waist portion that are overlaid in a thickness direction, in the step of forming the side-joining-portion region, the side joining portion being formed so that a center line of the side joining portion is shifted with respect to a center line of the welded portion pair that is adjacent to the side-joining-portion region in the direction of transport, when materials are viewed in the front-back direction.

According to such a method for manufacturing an underpants-shaped absorbent article, it is possible to reduce the probability that the protrusion pattern, which forms the side joining portions, overlaps the elastic member (area ratio). Therefore, the elastic member is less likely to be severed by the protrusion pattern, which forms the side joining portion, and the elastic member can be prevented from coming off from the side-joining-portion region.

A method for manufacturing an underpants-shaped absorbent article, the underpants-shaped absorbent article having a vertical direction, a lateral direction and a front-back direction that intersect each other, the underpants-shaped absorbent article including an absorbent main body and a waist portion in which a front waist portion and a back waist portion are joined by a pair of side-joining-portion regions in two ends in the lateral direction, the method including: a step of forming a continuous body of the front waist portion and a continuous body of the back waist portion, by forming a plurality of welded portion pairs when forming a plurality of welded portions after placing a plurality of continuous bodies of an elastic member between a skin-side-sheet continuous body and a non-skin-side-sheet continuous body, the plurality of continuous bodies of the elastic member being in a state of stretching in a direction of transport corresponding to the lateral direction, the plurality of continuous bodies of the elastic member being placed side-by-side in an intersecting direction of the direction of transport, the plurality of welded portions being portions that join the skin-side-sheet continuous body and the non-skin-side-sheet continuous body, the skin-side-sheet continuous body and the non-skin-side-sheet continuous body being continuous in the direction of transport, the plurality of welded portion pairs holding composed of the welded portions that are placed on both sides of each of the plurality of continuous bodies of the elastic member in the intersecting direction, when the elastic member in the underpants-shaped absorbent article contracts in the lateral direction, the plurality of welded portion pairs holding the elastic member and restricting a position of the elastic member in the lateral direction with respect to the skin-side sheet and the non-skin-side sheet; and a step of forming a side-joining-portion region by forming a plurality of side joining portions arranged side-by-side in the intersecting direction, the plurality of side joining portions being portions for joining the continuous body of the front waist portion and the continuous body of the back waist portion that are overlaid in a thickness direction, in the step of forming the side-joining-portion region, the side joining portion being formed while moving an intersecting-direction position of the continuous bodies of the elastic member that are in the state of stretching in the direction of transport.

According to such a method for manufacturing an underpants-shaped absorbent article, it is possible to push the elastic member out of the protrusion pattern, which forms the side joining portion. Therefore, the elastic member is less likely to be severed by the protrusion pattern, which forms the side joining portion, and the elastic member can be prevented from coming off from the side-joining-portion region.

In such a method for manufacturing an underpants-shaped absorbent article, in the step of forming the side-joining-portion region, the side joining portion is formed by the ultrasonic welding.

According to such a method for manufacturing an underpants-shaped absorbent article, the vibrations of the ultrasonic welding make the elastic member likely to move. Therefore, the elastic member is likely to be pushed out of the protrusion pattern, which forms the side joining portions (the position of the elastic member is likely to displace in the intersecting direction), and it is possible to suppress the cutting off of the elastic member.

### Embodiment

The following describes an underpants-shaped absorbent article according to the present invention by way of example of an underpants-shaped disposable diaper 1 for infants (hereinafter, also referred to as a "diaper"). However, the absorbent article according to the present invention is not limited thereto, and is also applicable to disposable diapers for adults, shorts-shaped sanitary napkins, and the like.

### Basic configuration of underpants-shaped disposable diaper 1

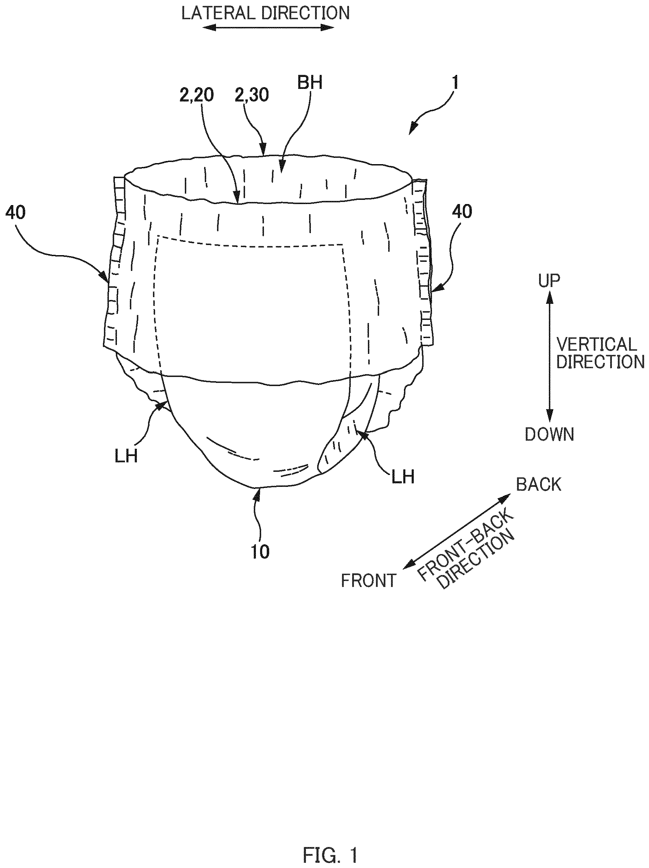

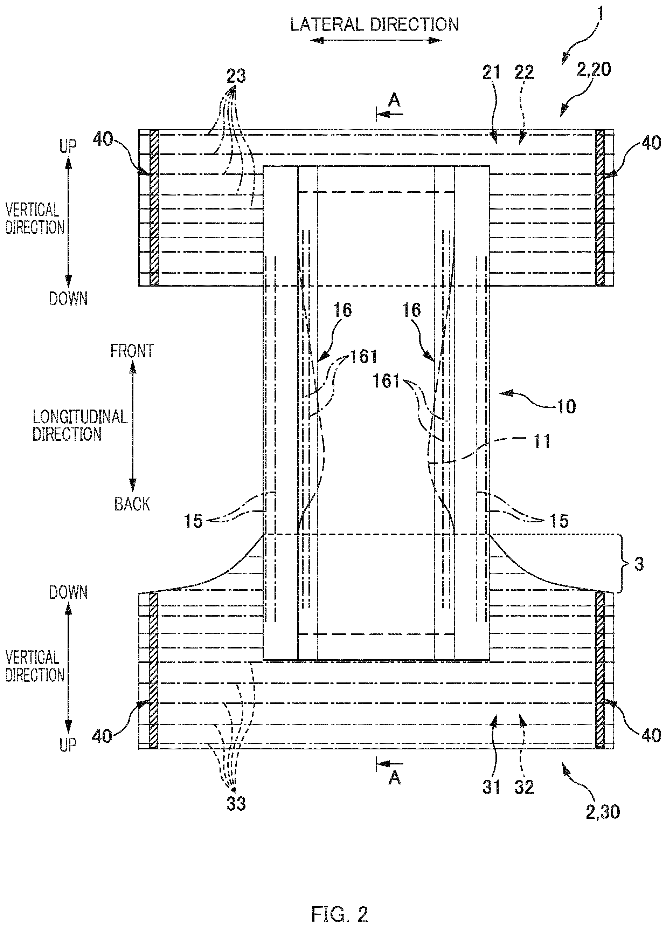

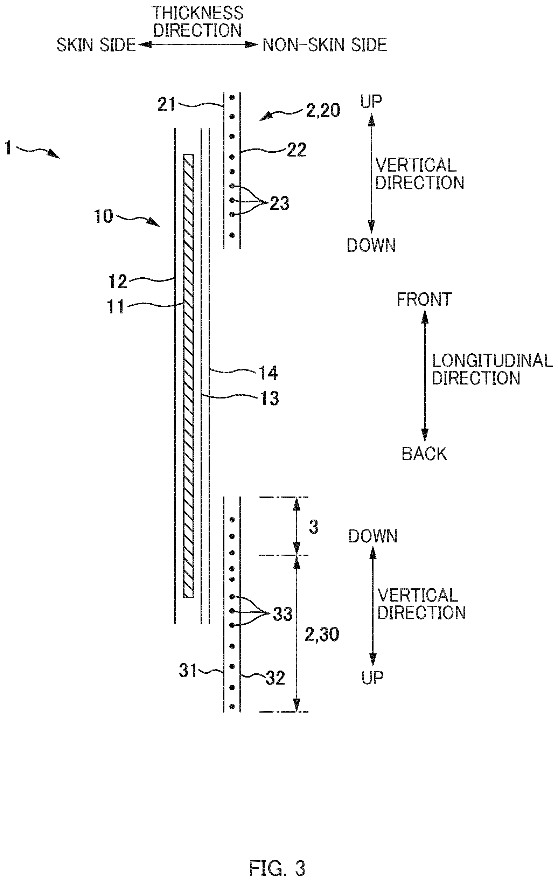

FIG. 1 is a schematic perspective view of the diaper 1. FIG. 2 is a schematic plan view of the diaper 1 in an unfolded and stretched state when viewed from a wearer's skin side. FIG. 3 is a schematic cross-sectional view taken along line A-A in FIG. 2.

The underpants-shaped diaper 1 has the vertical direction, the lateral direction, and the front-back direction that intersect each other, and has the waist opening BH and a pair of the leg openings LH. In the vertical direction, the side of the waist opening BH is the upper side, and the side of the wearer's crotch is the lower side. In the front-back direction, the side corresponding to the wearer's stomach side is the front side, and the side corresponding to the wearer's back side is the back side. Further, the diaper 1 has a thickness direction in which constituent members of the diaper 1 are overlaid on each other as shown in FIG. 3, and in the thickness direction, the side that comes into contact with the wearer refers to the skin side, and the side opposite to the skin side refers to the non-skin side.

The diaper 1 include: an absorbent main body 10; and a waist portion 2 in which a front waist portion 20 and a back waist portion 30 are joined by a pair of side-joining-portion regions 40 in two lateral ends. The waist portion 2 is arranged on the non-skin side of the absorbent main body 10, and is joined to the absorbent main body 10 with adhesive, etc.

In the diaper 1 of the present embodiment, the front waist portion 20 and the back waist portion 30 are constituted by separate members, and the non-skin-side surface of the absorbent main body 10 is exposed in the crotch portion. But the configuration is not limited to this. For example, a diaper may include an exterior member that constituted by three members, namely the front waist portion 20, the back waist portion 30, and the crotch portion connecting them. Or a diaper may include an exterior member that constituted by a single member that continues from the front waist portion 20 to the back waist portion 30.

As shown in FIG. 3, the absorbent main body 10 includes: an absorbent body 11; a liquid-permeable top sheet 12 that is arranged on the skin side with respect to the absorbent body 11; a liquid-impermeable back sheet 13 that is arranged on the non-skin side with respect to the absorbent body 11; and an exterior sheet 14. The absorbent core 11 needs to be any material that absorbs and retains excreted fluid, and examples thereof include those formed by molding liquid absorbent material such as pulp fiber and superabsorbent polymer into a predetermined shape. The absorbent core 11 may be covered with a liquid permeable sheet such as tissue paper, nonwoven fabric, or the like.

Further, as shown in FIG. 2, in the two lateral sides of the absorbent main body 10, leg elastic members 15 (e.g., elastic strings) may be provided, which stretches and contracts along the longitudinal direction of the absorbent main body 10. Furthermore, in the two lateral sides of the absorbent main body 10 (inside the leg elastic members 15), leak-proof wall portions 16 that can stand up toward the skin side may be provided. This makes it possible to suppress side leakage of excreted fluid. For example, the leak-proof wall portions 16 are formed as follows: the two lateral sides of the exterior sheet 14 are folded inward in the lateral direction so as to be located on the skin-side surface of the top sheet 12, and the leak-proof-wall elastic members (e.g., the elastic string) that stretch and contract in the longitudinal direction are provided in the folded portion.

The front waist portion 20 and the back waist portion 30 are rectangular members in plan view, and respectively have skin-side sheets 21 and 31, non-skin-side sheets 22 and 32, and a plurality of waist elastic members 23 and 33 (e.g., elastic strings) that stretch and contract in the lateral direction. The plurality of waist elastic members 23 (33) are arranged side by side in the vertical direction between the skin-side sheet 21 (31) and the non-skin-side sheet 22 (32).

As the skin-side sheets 21, 31 and the non-skin-side sheets 22, 32, flexible sheet members are preferable, and examples include spunbond nonwoven fabric, meltblown nonwoven fabric, and SMS (spunbond/meltblown/spunbond) nonwoven fabric. The constituent fibers of the nonwoven fabric are not limited to a single fiber formed of polypropylene (PP), which is a typical example of the thermoplastic resin. It is possible to use a single fiber formed of other thermoplastic resin such as polyethylene (PE), and furthermore, it is possible to use a composite fiber having a sheath-core structure of PE, PP and the like.

Also, like the present embodiment, the skin-side sheets 21, 31 and the non-skin-side sheets 22, 32 may be composed of two sheets, or may be formed by two layers of a sheet in which one sheet is folded back in the vertical direction and overlaid. Further, the waist portion may have another sheet that is overlaid on the skin-side sheet and the non-skin-side sheet, and three or more layers of these sheets may be joined by the later-described welded portions 50.

Furthermore, in the diaper 1, portions of the exterior member that overlap the side-joining-portion region 40 with respect to the vertical direction are the waist portion 2 (front waist portion 20 and back waist portion 30). The diaper 1 may have a portion of the exterior member that extends from the waist portion 2 toward the crotch side (in the present embodiment, a portion 3 extending from the back waist portion 30). This makes it possible to cover the wearer's buttocks and lower abdomen.

### Waist Portion 2

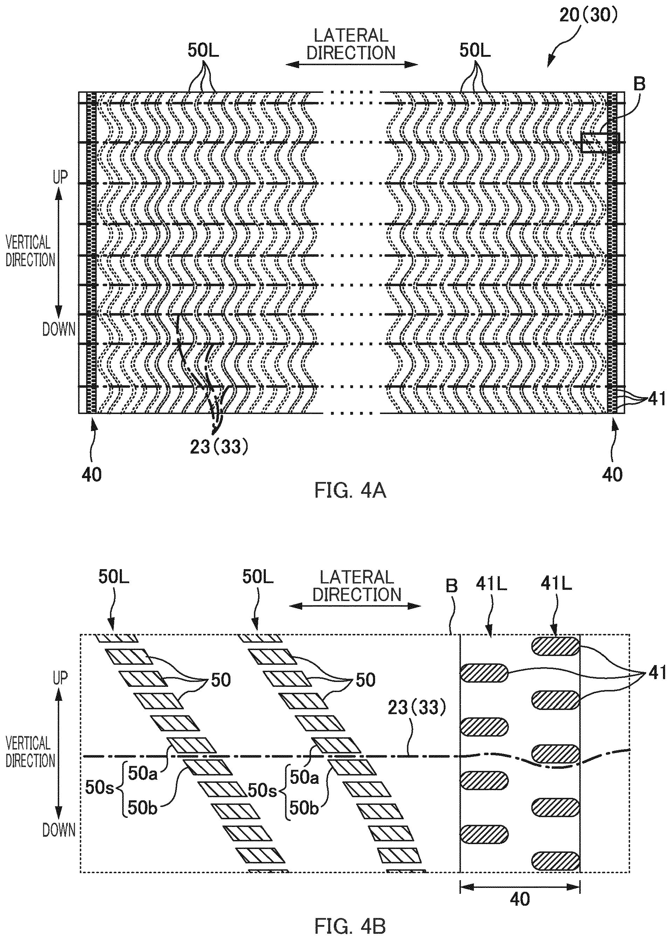

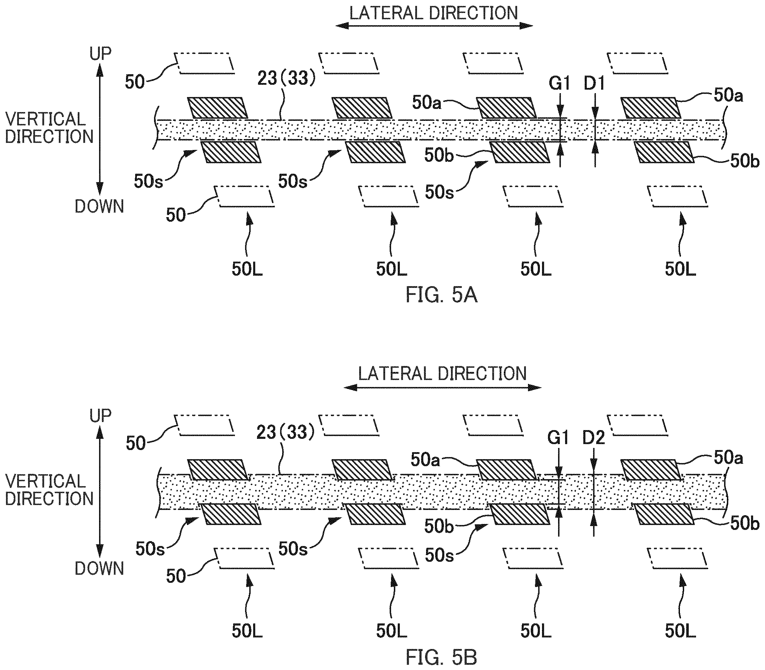

FIG. 4A is a diagram illustrating the side-joining-portion regions 40 and welded portion rows 50L provided in the waist portion 2, and FIG. 4B is a schematic enlarged view of the section B in FIG. 4A. FIGS. 5A and 5B are diagrams illustrating a function of attaching the waist elastic member 23 (33).

The skin-side sheet 21 and the non-skin-side sheet 22, which constitute the front waist portion 20, are intermittently joined by a plurality of the welded portions 50 that are discretely arranged in the vertical direction and the lateral direction. Similarly, the skin-side sheet 31 and the non-skin-side sheet 32, which constitute the back waist portion 30, are intermittently joined by a plurality of the welded portions 50 that are discretely arranged in the vertical direction and the lateral direction.

The front waist portion 20 and the back waist portion 30 have a substantially identical configuration. Therefore, when the contents described below is the same for both the front waist portion 31 and the back waist portion 41, only the front waist portion 31 will be described as a representative for both. Regarding the back waist portion 41, the reference signs of its components and the like corresponding to those of the front waist portion 31 will be indicated by blanketing as necessary.

As shown in FIGS. 4A and 4B, in the front waist portion 20 (30), a plurality of the welded portion rows 50L are arranged in the lateral direction at intervals, each of the welded portion rows 50L constituted by a plurality of the welded portions 50 are arranged in the vertical direction at intervals. FIG. 4A shows the welded portion rows 50L extending in the vertical direction while meandering left and right in a curved manner, but the shape of the welded portion rows 50L is not particularly limited. For example, it may be a row having a shape that extends straight along the vertical direction, or may be a row having a straight-line- shape or curved shape that is inclined toward the one side in the lateral direction.

The waist elastic member 23 (33) is arranged so as to pass between the welded portions 50 that is lined up in the vertical direction, as shown in FIG. 4B. For this purpose, it is recommended to align the vertical positions of the welded portions 50, which are lined up ins the lateral direction. This makes aligned the vertical positions of the spaces where a waist elastic member 23 (33) passes and where are located between the welded portions 50 adjacent in the vertical direction, and this makes it possible to arrange the waist elastic member 23 along the lateral direction.

In the following description, among the plurality of the welded portions 50, the two welded portions 50 that are located on both sides of the waist elastic member 23 (33) in the vertical direction are correctively referred to as a "welded portion pair 50S". Of the welded portion pair 50S, the welded portion 50 arranged above the waist elastic member 23 (33) is referred to as an "upper welded portion 50a", and the welded portion 50 arranged below the waist elastic member 23 (33) is referred to as a "lower welded portion 50b".

It is preferable that the waist elastic member 23 (33) passes in the lateral direction between multiple welded portion pairs 50S whose positions in the vertical direction are the same, but the waist elastic member 23 (33) may pass between the welded portion pairs 50S whose positions in the vertical direction are different. In this case as well, it is possible to give the waist portion 20 the stretchability in the lateral direction.

Further, as shown in FIG. 5A, the upper welded portion 50a and the lower welded portion 50b, which form the welded portion pair 50S, are lined up with a gap G1 in the vertical direction. The size of the gap G1 is set to be equal to or slightly larger than the outer diameter D1 of the waist elastic member 23 (33) that is in a state where the waist portion 20 stretchs to the maximum extent in the lateral direction (G1 ≥ D1).

However, as the waist elastic member 23 (33) contracts in the lateral direction, it becomes thicker. For this purpose, as shown in FIG. 5B, the gap G1 of the welded portion pair 50S is set to be smaller than the maximum external dimension D2 of the waist elastic member 23 (33) that is in a state where the waist elastic member 23 (33) contracts in the lateral direction (that is, the natural state of the waist portion 20) (G1 < D2). Accordingly, the waist elastic member 23 (33) in a state of contracting in the lateral direction is sandwiched in the vertical direction between the welded portion pair 50 (the expansion in the vertical direction is restricted). Therefore, the position (movement) of the waist elastic member 23 (33) in the lateral direction and the vertical direction with respect to the skin-side sheet 21 (31) and the non-skin-side sheet 22 (33) is restricted.

Therefore, even if the waist elastic member 23 (33) is not fixed using an adhesive for the skin-side sheet 21 (31) and the non-skin-side sheet 22 (32), it is possible to suppress the displacement of the waist elastic member 23 (33), making possible to maintain the stretchability of the waist portion 2. In other words, in the diaper 1 of the present embodiment, using the welded portions makes it possible to attach the waist elastic member 23 (33) to the skin-side sheet 21 (31) and the non-skin-side sheet 22 (33) without using the adhesive or with reducing the amount of adhesive. Therefore, it is possible to suppress the deterioration of the flexibility in the waist portion 20 due to the hardening of adhesive. Further, it is possible to suppress the deterioration in the elastic characteristics of the waist elastic member 23 (33) due to hardening of adhesive.

### Suppression of coming off and severing of the waist elastic members 23 and 33

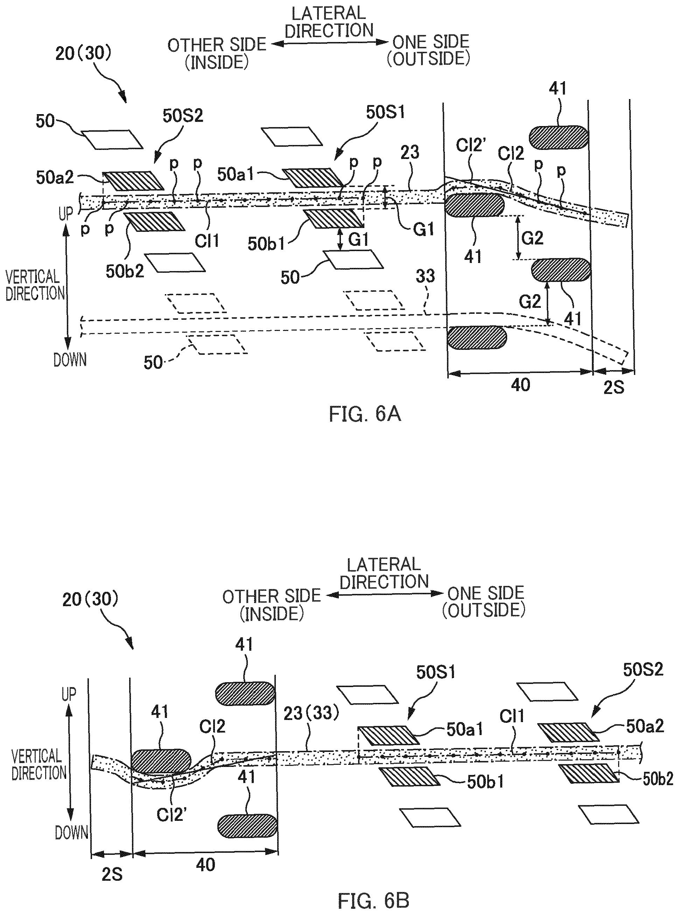

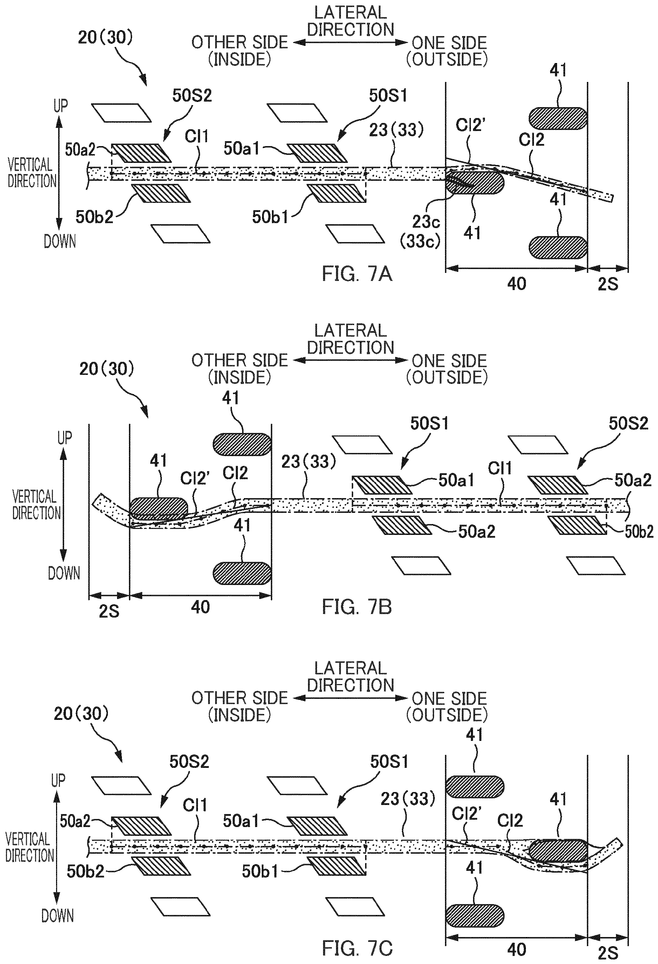

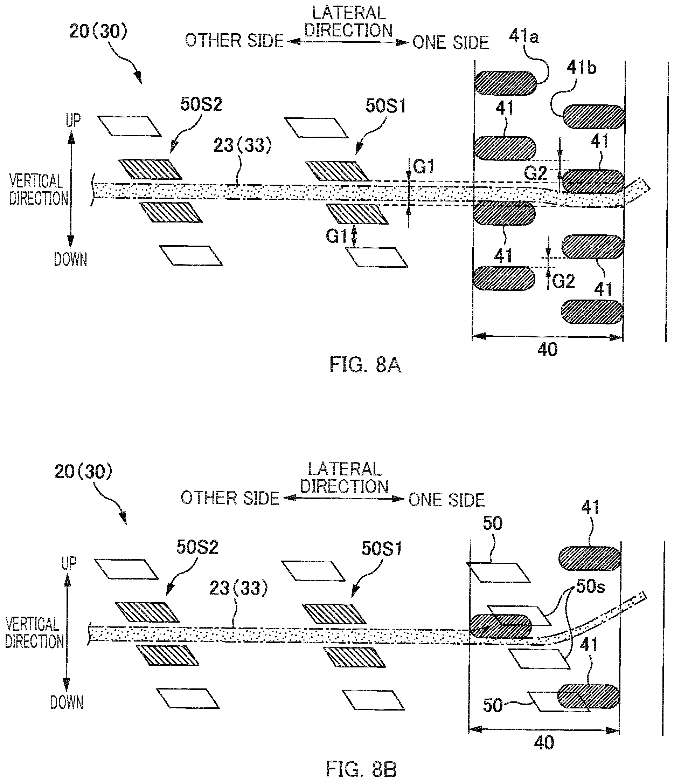

FIGS. 6A and 6B are diagrams showing a state in which an end portion of the waist elastic member 23 (33) is in contact with the side joining portion 41. FIGS. 7A to 7C are diagrams showing a state in which the end portion of the waist elastic member 23 (33) is sandwiched on the side joining portion 41. FIGS. 8A and 8B are diagrams showing modified examples of the side-joining-portion region 40.

The front waist portion 20 and the back waist portion 30 are joined by a pair of the side-joining-portion regions 40 in the both ends in the lateral direction. As shown in FIGS. 4A and 4B, in the side-joining-portion region 40, a plurality of the side joining portions 41 are arranged side by side in the vertical direction. Examples of a method for forming the side joining portions 41 include welding means such as ultrasonic welding, heat welding, and high-frequency sealing, and non-heating pressurizing means.

As shown in FIG. 2, the waist elastic members 23 (33) are provided extending along the lateral direction from the side-joining-portion region 40 on the lateral one side to the side-joining-portion region 40 on the lateral other side. Further, in order to prevent the front waist portion 20 and the back waist portion 30 from separating during usage of the diaper 1, the sheet which constitutes the waist portion 2 is firmly joined (compressed) in the side-joining-portion regions 40. Therefore, the lateral end portions of the waist elastic members 23 (33) are firmly sandwiched and fixed between the skin-side sheet 21 (31) and the non-skin-side sheet 22 (32) in the side-joining-portion regions 40. Therefore, in the side-joining-portion regions 40, the stretchability of the waist elastic members 23 (33) does not exhibit, and the stretchability of the waist elastic members 23 (33) exhibits between the pair of the side-joining-portion regions 40.

However, in the diaper 1 of the present embodiment, the displacement of the waist elastic members 23 (33) is restricted using the welded portion pairs 50S, which hold the waist elastic members 23 (33) inbetween in the vertical direction. Accordingly, the waist elastic member 23 (33) are attached to the sheet without using an adhesive, or with a small amount of an adhesive. Therefore, if the waist elastic member 23 (33) comes off from the side-joining-portion region 40, there is a risk that the waist elastic member 23 (33) further comes off from the welded portion pair 50S, which is lined up in the lateral direction. In this case, the waist elastic member 23 (33) cannot give the stretchability to the waist portion 2, and the stretchability of the waist portion 2 decreases.

Therefore, in the diaper 1 of the present embodiment, at least one (first elastic member) of the plurality of waist elastic members 23 (33) included in the diaper 1 satisfies the following conditions. First, as shown in FIG. 6A and the like, among the plurality of welded portion pairs 50S, the welded portion pair 50S which is located adjacent to and laterally inside the side-joining-portion region 40 that is located on the one side (or the other side) in the lateral direction and between which the waist elastic member 23 (33) is sandwiched is referred to as a "first welded portion pair 50S1". Furthermore, the welded portion pair 50S, which is adjacent to and laterally inside the first welded portion pair and between which the same waist elastic member 23 (33) is sandwiched is referred to as a "second welded portion pair 50S2".

When the diaper 1 (waist portion 2) in the stretched state is viewed in the front-back direction (thickness direction), the center line Cl2 of a portion of the waist elastic member 23 (33) that is located in the side-joining-portion region 40 located on the one side (or the other side) in the lateral direction, is inclined with respect to the center line Cl1 of a portion of the same waist elastic member 23 (33) that is located between the first welded portion pair 50S1 and the second welded portion pair 50S2.

The center lines Cl1 and Cl2 of the waist elastic member 23 (33) are center lines that divide the waist elastic member 23 (33) in the vertical direction. In addition, the portion of the waist elastic member 23 (33) that is located between the first welded portion pair 50S1 and the second welded portion pair 50S2 is a portion of the waist elastic member 23 (33) that is located between the lateral outermost end of the first welded portion pair 50S1 and the lateral innermost end of the second welded portion pair 50S2.

The portion of the waist elastic member 23 (33) that is located between the first welded portion pair 50S1 and the second welded portion pair 50S2 is arranged extending along the lateral direction and exhibits the stretchability in the lateral direction. Therefore, the center line Cl1 substantially conforms to the line extending along the lateral direction. On the other hand, in the side-joining-portion region 40, the end of the waist elastic member 23 (33) that has been cut off during the manufacture of the diaper 1 is sandwiched and fixed between the skin-side sheet 21 (31) and the non-skin side sheet 22 (32), and the stretchability does not exhibit. Therefore, the center line Cl2 can be inclined with respect to the center line Cl1 (with respect to the lateral direction).

Further, the location of the waist elastic member 23 (33) in the side-joining-portion region 40 is not restricted by the welded portion pair 50S. Therefore, even in a state where the waist portion 20 is stretched, the probability that the center line Cl2 has a curved shape as shown in FIG. 6A and the like is high. if it has been visually conformed the center lined that at least a part of the center line Cl2 is inclined with respect to the center line Cl1 (lateral direction), the center line Cl2 is inclined with respect to the center line Cl1.

More rigorous confirmation methods include the following methods. First, an enlarged image of the waist portion 2 in the stretched state is taken using a digital microscope or the like. On the captured image data, the center points p of the target waist elastic member 23 (33) in the vertical direction is specified at predetermined intervals in the lateral direction. A straight line connecting the specified plurality of center points p or a straight line approximated by the method of least squares is obtained as the center lines Cl1 and Cl2', and the slopes thereof are compared.

Note that the stretched state of the diaper 1 is a state where the elastic members included in the diaper 1 have been stretched to such an extent that the wrinkles of the diaper 1 are no longer visible. Specifically, these elastic members are stretched until the dimensions of constituent members of the diaper 1 match or are close to the dimensions of the members on their own (i.e., the dimensions in a state where the stretchability of the elastic members is not exhibited). In addition, when confirming the slopes of the center lines Cl1 and Cl2, the confirmation may be made in a state where, by releasing the joining of the side-joining-portion region 40, the diaper 1 is opened and unfolded flat (FIG. 2), or in a state where the front waist portion 20 and the back waist portion 30 are connected.

The direction to which the center line Cl1 of the waist elastic member 23 (33) located between the first welded portion pair 50S1 and the second welded portion pair 50S2 conforms (lateral direction) is the direction in which the contractive force of the waist elastic member 23 (33) acts. In other words, this is the direction in which a force that causes the waist elastic member 23 (33) to come off from the side-joining-portion region 40 (so-called rubber coming off) acts. Therefore, by inclining the center line Cl2 of the waist elastic member 23 (33) in the side-joining-portion region 40 with respect to the center line Cl1 (lateral direction), it is possible to prevent a force that causes rubber coming off from directly acting on a portion of the waist elastic member 23 (33) that is located in the side-joining-portion region 40 (it is possible to disperse a force that causes rubber coming off). Therefore, it is possible to suppress the coming off the waist elastic member 23 from the side-joining-portion region 40.

Further, in the case where the waist elastic member 23 (33) located in the side-joining-portion region 40 is inclined with respect to the lateral direction, compared to the case where the waist elastic member 23 (33) extend conforming to the lateral direction, the length of a portion of the waist elastic member 23 (33) that is located in the side-joining-portion region 40 is longer. As mentioned above, in the side-joining-portion region 40, compared to other regions, the waist elastic member 23 (33) is more tightly sandwiched between sheets and the frictional force with the sheets is higher. Therefore, by increasing the length of the portion of the waist elastic member 23 (33) that is located in the side-joining-portion region 40, it increases the length of a portion of the waist elastic member 23 (33) on which the frictional force with the sheets is strongly exerted. This makes it possible to suppress the waist elastic member 23 (33) from coming off from the side-joining-portion region 40.

Therefore, in the case where the end portion 2S of the waist portion 2 exists outside the side-joining-portion region 40 in the lateral direction, as shown in FIG. 6A and the like, it is preferable that the waist elastic member 23 (33) extends laterally outside the side-joining-portion region 40 located on the one side (or other side) in the lateral direction (up to the end portion 2S).

Therefore, the waist elastic member 23 (33) extend over the entire region of the side-joining-portion region 40 in the lateral direction, where the frictional force with the sheets is strong. Therefore, the length of the portion of the waist elastic member 23 (33) on which the frictional force with the sheets is strongly exerted becomes longer, and it is possible to further suppress the coming off of the waist elastic member 23 (33) from the side-joining-portion region 40. However, the present invention is not limited to the above, and the waist elastic member 23 (33) may be interrupted in the middle of the side-joining-portion region 40.

According to such a diaper 1 of the present embodiment, the welded portion pairs 50S reduces the amount of an adhesive with which the waist elastic members 23 (33) are attached. In addition, it maintains the flexibility of the waist portion 2, and suppresses the coming off of the waist elastic members 23 (33), maintaining the stretchability of the waist portion 2.

In addition, in order to make the waist elastic member 23 (33) be inclined in the side-joining-portion region 40, for example, as shown in FIG. 6A (or FIG. 6B), it is preferable that a part of the waist elastic member 23 (33) is in contact with the side joining portion 41 in the side-joining-portion region 40 on the one side (or the other side) in the lateral direction.

By doing so, the waist elastic member 23 (33) conforms to the outline of the side joining portion 41, and this can make the center line Cl2 of the waist elastic member 23 (33) located in the side-joining-portion region 40 be inclined with respect to the center line Cl1 (the lateral direction). In this case, since the frictional force occurs at the location where the waist elastic member 23 (33) and the side joining portion 41 come into contact, it is possible to further suppress the coming off of the waist elastic member 23 (33) from the side-joining-portion region 40.

Alternatively, as shown in FIGS. 7A to 7C, in the side-joining-portion region 40 located on the one side (or the other side) in the lateral direction, a part of the waist elastic member 23 (33) may overlap the side joining portion 41 in the thickness direction. In other words, a part of the waist elastic member 23 (33) may be sandwiched between the skin-side sheet 21 (31) and the non-skin-side sheet 22 (33) by the side joining portions 41.

In this case as well, portions of the waist elastic member 23 (33) that are not sandwiched by the side joining portion 41 conforms to the outline of the side joining portion 41, and this can make the center line Cl2 of the waist elastic member 23 (33) located in the side-joining-portion region 40 be inclined with respect to the center line Cl1 (the lateral direction). In this case, a part of the waist elastic member 23 (33) in the side joining portion 41 is strongly sandwiched between the sheets, and this makes it possible to further suppress the waist elastic member 23 (33) from coming off from the side-joining-portion region 40.

As shown in FIG. 7A, even if a part of the waist elastic member 23 (33) is cut off in the middle in the lateral direction, it is sufficient that the cut end portion 23c (33c) is fixed at the side joining portion 41. In this case as well, it is possible to suppress the coming off of the waist elastic member 23 (33) from the side-joining-portion region 40.

Further, if a part of the waist elastic member 23 (33) overlap the side joining portion 41 in the thickness direction (see FIG. 7A), it is preferable to specify the center line Cl2 in a portion of the waist elastic member 23 (33) that does not overlap the side joining portion 41. Further, in the case where the waist elastic member 23 (33) branches at the side joining portion 41 (see FIG. 7C), it is preferable to specify the center line Cl2 in a portion of the waist elastic member 23 (33) that has a larger area.

Further, it is preferable that the waist elastic member 23 (33) is is inclined with respect to the center line Cl1 (the lateral direction), in the side-joining-portion region 40 on both lateral sides. This makes the waist elastic member 23 (33) less likely to come off from the side-joining-portion region 40 on both lateral sides. Therefore, the waist elastic member 23 (33) can give the stretchability to the waist portion 2 throughout between the pair of the side-joining-portion regions 40.

Preferably, the waist elastic member 23 partially overlaps in the thickness direction the side joining portion 41 located on the one side in the lateral direction, and partially is in contact with the side joining portion 41 located on the other side in the lateral direction. Alternatively, a part of the waist elastic member 23 (33) may overlap the side joining portion 41 in the thickness direction, on both sides in the lateral direction. Or a part of the waist elastic member 23 (33) may be in contact with the side joining portion 41, on both sides in the lateral direction. In these cases, the waist elastic member 23 (33) is less likely to come off from the side-joining-portion region 40 on both lateral sides.

Further, it is preferable that a larger number of the waist elastic members 23 (33) included in the diaper 1 are inclined with respect to the center line Cl1 (the lateral direction), in the side-joining-portion regions 40. This makes it possible to suppress the coming off of the waist elastic members 23 (33) from the side-joining-portion region 40, and making it possible to maintain the stretchability of the waist portion 2.

Preferably, in the side-joining-portion region 40 on the one side in the lateral direction, there is one or more waist elastic members 23 (33) (first elastic member) that partially overlap the side joining portion 41 in the thickness direction, and there is one or more waist elastic members 23 (33) (second elastic member) that are partially in contact with the side joining portion 41. This makes it possible to further suppress the coming off of the plurality of waist elastic members 23 (33) from the side-joining-portion region 40.

Further, as shown in FIG. 6A, it is preferable that the gap G2 in the vertical direction between the side joining portions 41 that are located beside the first welded portion pair 50S1 in the lateral direction is equal to or greater than the gap G1 of the first welded portion pair 50S1 in the vertical direction (G2 ≥ G1) .

Note that the gap G1 of the first welded portion pair 50S1 in the vertical direction is the length in the vertical direction from the lower end of the upper welded portion 50a1 to the upper end of the lower welded portion 50b1. Similarly, the gap G2 in the vertical direction between the side joining portions 41 is the length in the vertical direction from the lower end of the side joining portion 41 on the upper side to the upper end of the side joining portion 41 on the lower side. In the case where the side joining portions 41 are arranged in a staggered manner as shown in FIG. 6A, it is a gap between the side joining portions 41 that are shifted in the lateral direction and are adjacent in the vertical direction. Furthermore, although the side joining portions 41 according to the present embodiment are lined up in the vertical direction with a constant gap G2, the gap G2 in the vertical direction between the side joining portions 41 may vary depending on the position in the vertical direction. Therefore, the gap G2 between the side joining portions 41 that are located beside the first welded portion pair 50S1 in the lateral direction (that is, the side joining portion 41 where its position in the vertical direction overlap that of the first welded portion pair 50S1, or the side joining portion 41 where its position in the vertical direction is closest to the first welded portion pair 50S1) is used as a comparison target.

By making the gap G2 in the vertical direction between the side joining portions 41 relatively large, it is possible to reduce the number of the side joining portions 40, which have a high stiffness. Therefore, it is possible to prevent the flexibility of the waist portion 2 from deteriorating due to the side-joining-portion regions 40. Furthermore, multiple side joining portions 41 are not be arranged between the first welded portion pair 50S1, that is, in a region that overlaps with respect to the vertical direction the space where the waist elastic member 23 (33) passes. Therefore, it is possible to reduce the probability that the waist elastic member 23 (33) overlaps the side joining portion 41 in the thickness direction (area ratio). Accordingly, when forming the side joining portions 41 by welding or pressurizing, it is possible to prevent the waist elastic members 23 (33) from being cut by a protrusion pattern that forms the side joining portions 41 (e.g., protrusions formed on the outer circumferential surface of rollers between sheets are sandwiched). Therefore, it is possible to suppress the cutting off of the waist elastic member 23 (33) from the side-joining-portion region 40.

However, contrary to the above, as shown in the modified example of FIG. 8A, the gap G2 in the vertical direction between the side joining portions 41 that are located beside the first welded portion pair 50S1 in the lateral direction may be shorter than the gap G1 of the first welded portion pair 50S1 in the vertical direction (G2 < G1).

In this case, a plurality of the side joining portions 41 (partially) are likely to be arranged between the first welded portion pairs 50S1, that is, in a region that overlaps with respect to the vertical direction the space where the waist elastic member 23 (33) passes. Therefore, a part of the waist elastic member 23 (33) is more likely to come into contact with the side joining portion 41 (FIG. 6A, etc.), or more likely to overlap the side joining portion 41 in the thickness direction (FIG. 7A, etc.). This makes it possible to further suppress the coming off of the waist elastic members 23 (33) from the side-joining-portion region 40.

In addition, as shown in FIG. 4B, in the side-joining-portion region 40, in the case where a plurality of rows 41L of the side joining portion in which a plurality of the side joining portions 41 are lined up in the vertical direction are lined up side-by-side in the lateral direction, it is preferable that the rows 41L composed of the plurality of side joining portions 41 are spaced apart in the lateral direction.

This makes it possible to increase the lateral length of the side-joining-portion region 40, where the frictional force of the waist elastic member 23 (33) with the sheets increases. Therefore, the length of a portion of the waist elastic member 23 (33) on which the frictional force with the sheets strongly acts becomes longer, and this makes it possible to further suppress the coming off of the waist elastic member 23 (33) from the side-joining-portion region 40. Further, the density (number/mm²) of the side joining portions 41 in the side-joining-portion region 40 can be reduced, and the flexibility in the waist portion 2 can be maintained.

In addition, in FIG. 4B, the side joining portions 41 are arranged in a staggered manner (that is, the side joining portions 41 included in each of two side-joining-portion rows 41L that are adjacent in the lateral direction are shifted in the vertical direction). However, the present invention is not limited to this, and the vertical positions of the side joining portions 41 adjacent in the lateral direction may be aligned. Further, the number of the side-joining-portion rows 41L may be one, or may be three or more.

Further, in the case where the side joining portions 41 are arranged in a staggered manner (FIG. 8A), the following is preferable. The distance G2 in the vertical direction between the side joining portions 41 that are located beside the first welded portion pair 50S1 in the lateral direction (in other words, the distance G2 in the vertical direction between the followings: a side joining portion 41 which is adjacent to the first welded portion pair 50S1 in the lateral direction; and a side joining portion 41 which is adjacent to the foregoing side joining portion 41 in the lateral direction and which is shifted with respect to the side joining portion 41 in the vertical direction) is smaller than the diameter of the waist elastic member 23 (33) in a state of contracting in the lateral direction (in other words, the width of the waist elastic member 23 in the vertical direction of diaper 1). The diameter of the waist elastic member 23 (33) in the state of contracting in the lateral direction is the diameter of the standalone waist elastic member 23 (33) that is separated from the diaper 1 and that is in the natural state, and as shown in FIG. 5B, is the maximum diameter D2 of the waist elastic member 23 (33) of the diaper 1 that is in the natural state.

By doing so, when fixing the waist elastic member 23 (33) that has been cut during the manufacture of the diaper 1 and is contracting, at the side joining portions 41, the waist elastic member 23 is sandwiched in the vertical direction between the side joining portions 41 whose diameter is smaller than that of the waist elastic member 23 (33). Therefore, the waist elastic member 23 comes into contact with at least one of the side joining portions 41 that is shifted with respect to the vertical direction and is lined up in the lateral direction, and this makes the waist elastic member 23 likely to bend along the contour of the side joining portion 41. Therefore, the center line Cl2 of the waist elastic member 23 (33) located in the side-joining-portion region 40 is inclined with respect to the center line Cl (the lateral direction), and it is possible to further suppress the coming off of the waist elastic member 23 from the side-joining-portion region 40.

Further, as shown in FIG. 8B, the welded portion 50 which joins the skin-side sheet 21 (22) and the non-skin-side sheet 22 (32) may be formed in the side-joining-portion region 40 as well. In that case, it is preferable that the side-joining-portion region 40 has a portion where the welded portion 50 and the side joining portion 41 overlaps in the thickness direction of the waist portion 2.

This makes it likely to form the side joining portion 41 so as to overlap in the thickness direction the waist elastic member 23 that passes between the welded portion pair 50S. Therefore, a part of the waist elastic member 23 (33) is more likely to come into contact with the side joining portion 41 (FIG. 6A, etc.), or more likely to overlap the side joining portion 41 in the thickness direction (FIG. 7A, etc.). This makes it possible to further suppress the coming off of the waist elastic members 23 (33) from the side-joining-portion region 40.

Furthermore, it is preferable that, in at least either one region of the pair of the side-joining-portion regions 40, as shown in FIG. 6A, at least one the waist elastic member 23 included in the front waist portion 20 is placed shifted in the vertical direction with respect to the waist elastic member 33 included in the back waist portion 30.

Accordingly, when forming the side joining portion 41 by welding or pressurization, it is possible to prevent both of the waist elastic members 23 on the front side and the waist elastic members 33 on the back side from being cut off by a protrusion pattern that forms the side joining portions 41. Therefore, it is possible to reduce the number of the waist elastic members 23 (33) that are cut by the protrusion pattern that forms the side joining portions 41, making it possible to maintain the stretchability of the waist portion 2.

Furthermore, by shifting the waist elastic member 23 on the front side with respect to the waist elastic member 33 on the back side in the vertical direction, it reduces the number of materials that overlap in the thickness direction in a portion where the side joining portions 41 are formed. Therefore, the skin-side sheets 21 and 31 and the non-skin-side sheets 22 and 32 can be firmly joined (compressed). By increasing the joining strength of the side joining portion 41, the frictional force between the waist elastic member 23 (33) and the sheets that are located in the side-joining-portion region 40 also increases, making it possible to further suppress the coming off of the waist elastic member 23 (33) from the side-joining-portion region 40.

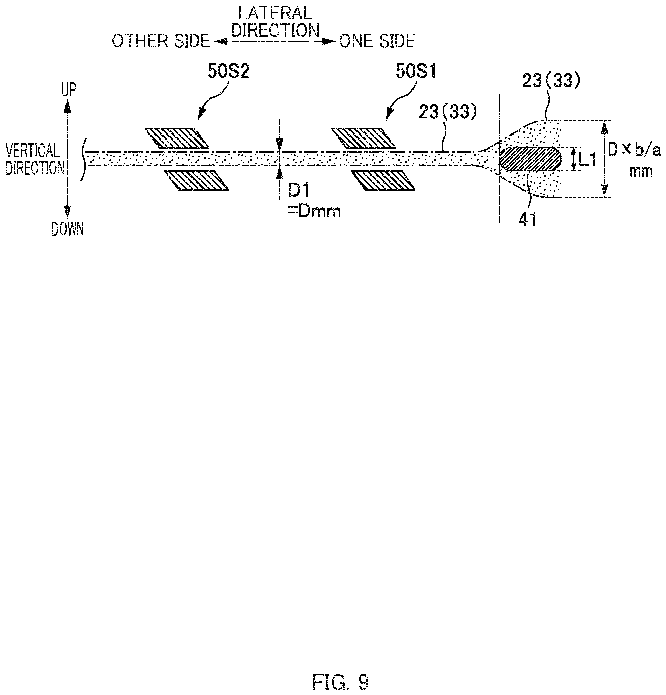

FIG. 9 is a diagram illustrating a length L1 of the side joining portion 41 in the vertical direction. Here, when the diaper 1 is stretched to the maximum in the lateral direction, the stretch factor of the waist elastic member 23 is referred to as a, and at this time, the width of the waist elastic member 23 in the vertical direction of the diaper 1 is D mm (= D1 in FIG. 5A). On the other hand, the maximum stretch factor of the standalone waist elastic member 23 (33) separated from the diaper 1, that is, the stretch factor immediately before the standalone waist elastic member 23 (33) is severed is referred to as b. Note that the stretch factor is the value obtained by dividing the length of the waist elastic member 23 (33) in the stretched state by the length of the waist elastic member 23 (33) in the natural state (no-load state).

Further, it is considered that the waist elastic member 23 (33) can stretch in the width direction (vertical direction of the diaper 1) to the stretch factor, which is similar to the longitudinal direction (lateral direction of the diaper 1). Therefore, the waist elastic member 23 (33) in the state arranged in the diaper 1, that is, the waist elastic member 23 (33) in the state where the stretch factor in the longitudinal direction (lateral direction) is a times and the width (length in the vertical direction) is D mm is considered to be capable of stretching in the width direction (vertical direction) up to b times. At this time, the width of the waist elastic member 23 (33) (length in the vertical direction) is considered to be "D × *b*/*a* (mm)".

Therefore, the vertical length L1 of the side joining portion 41 that is located beside the first welded portion pair 50S1 in the lateral direction is set to be equal to or less than "D × *b*/*a* (mm)". For example, if the stretch factor a of the waist elastic member 23 at the time when the diaper 1 stretches to the maximum is 2.5 times, the width D mm is 0.2 mm, and the maximum stretch factor b of the waist elastic member 23 is 8 times, then the vertical length L1 of the side joining portion 41 is preferably 0.6 mm (= 0.2 × 8/2.5) or less.

By doing so, even if the side joining portion 41 is formed overlapping the waist elastic member 23 (33), the vertical length of the waist elastic member 23 (33) is longer than the vertical length L1 of the side joining portion 41, and this increases the probability that the waist elastic member 23 (33) extends to the vertical direction without being cut off. Therefore, it is possible to prevent the waist elastic member 23 (33) from being cut off by being crushed and severed by the protrusion pattern, which forms the side joining portion 41. Therefore, it is possible to suppress the cutting off of the waist elastic member 23 (33) from the side-joining-portion region 40.

In addition, in the case where the cross-sectional shape of the waist elastic member 23 (33) is a circle, the width D mm of the waist elastic member 23 in the vertical direction of the diaper 1 corresponds to the diameter of the waist elastic member 23 (33). However, the waist elastic members 23 (33) may be ones having a cross-sectional shape other than a circle (e.g., an oval shape, etc.) or ones having a flat cross-section, so-called flat rubber.

Further, the stretch factor a times, b times, and width D mm can be measured by well-known methods. For example, measure the lateral length WO of the front waist portion 20 (30) in the natural state of the diaper 1, and measure the lateral length W1 of the front waist portion 20 (30) when being stretched in the lateral direction to the maximum. Then, the value obtained by dividing the length W1 when being stretched by the length WO at the natural state (W1/WO) is preferably set as a times the value (W1/WO). Further, in a state where the front waist portion 20 (30) is stretched to the maximum extent in the lateral direction, it is preferable to measure the vertical width D mm of the waist elastic member 23 (33) through the sheet that constitutes the front waist portion 20 (30). Alternatively, the width D mm in the direction corresponding to the vertical direction of the diaper 1 may be measured by taking out the waist elastic member 23 (33) from the front waist portion 20 (30) and stretching it a times. In addition, the waist elastic member 23 (33) taken out from the front waist portion 20 (30) is set between chucks of a tensile tester, and is made stretched in the stretching direction of the waist elastic member 23 (33), and obtaining a load at the time of cutting the waist elastic member 23 (33). Measure the length L0 of another the waist elastic member 23 (33) taken out from the front waist portion 20 (30) at the natural state. Thereafter, the waist elastic member 23 (33) is set in a tensile tester and is stretched to a load slightly smaller than the load at the time of cutting, and obtaining the length L1 of the waist elastic member 23 (33) based on the distance between the chucks at that time. It is preferable that the value obtained by dividing the length L1 by the length L0 of the natural state (L1/L0) is multiplied by b times.

Further, as shown in FIG. 4B, the side joining portion 41 of the present embodiment is illustrated as a quadrilateral with rounded corners, but the shape of the side joining portion 41 is not particularly limited. However, like the side joining portion 41 shown in FIG. 4B, it is preferable that the side joining portion 41 has a curved portion in at least a part of its peripheral portion. Examples of other shapes having a curved portion include a circular shape, an elliptical shape, a shape having an uneven outline, and the like. In this case, the waist elastic member 23 (33) is likely to conform to the outline of the side joining portion 41 (the curved portion), and the center line Cl2 of a portion of the waist elastic member 23 (33) that is located in the side-joining-portion region 40 is becomes likely to be inclined toward the center line Cl1 of a portion of the waist elastic member 23 that is located between the first welded portion pair 50S1 and the second welded portion pair 50S2 (lateral direction). Therefore, the coming off of the waist elastic member 23 (33) from the side-joining-portion region 40 can be further suppressed. Note that the shapes of all the side joining portions 41 that are arranged in the side-joining-portion region 40 are not limited to the same shape, and the shapes of the side joining portions 41 may differ depending on the location in the vertical direction, for example.

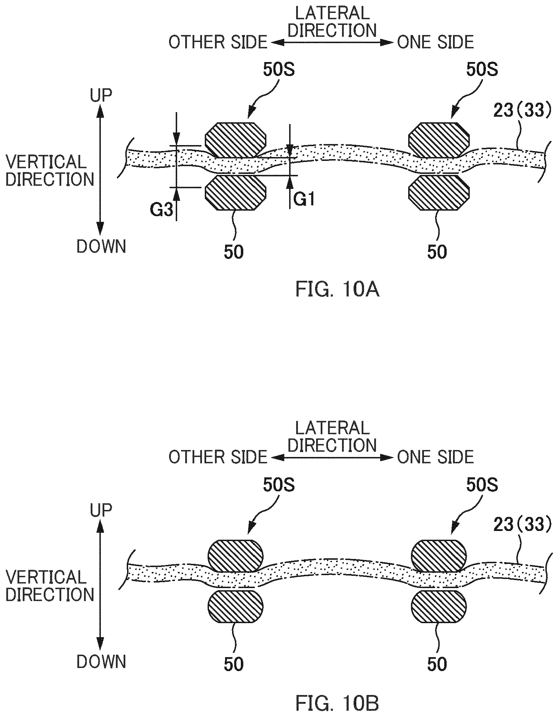

FIGS. 10A and 10B are diagrams illustrating modified examples of the welded portions 50. In FIG. 4B, the welded portions 50 having a parallelogram shape are illustrated, but the shape of the welded portions 50 is not particularly limited. For example, the welded portions 50 may have a shape in which, as shown in FIG. 10A, the distance in the vertical direction between two welded portions 50 that constitute the welded portion pair 50S is wider in at least an end portion located on a one side in the lateral direction (in FIG. 10A, both end portions G3) than in the central portion of the welded portion pair 50S in the lateral direction (G1).

In this case, the waist elastic member 23 can move in the vertical direction in the lateral end portions of the welded portions 50, where the gap (G3) is wide, while extending in the lateral direction between the welded portion pairs 50S that are lined up in the lateral direction. That is, along the lateral end portions of the welded portions 50, the waist elastic member 23 can be slightly inclined with respect to the lateral direction. This makes it the waist elastic member 23 (33) less likely to come off from the welded portion pair 50S, and the stretchability of the waist portion 2 is maintained.

In addition, as shown in FIG. 10A, in the case where the gap G3 of the both lateral end portions in the welded portion pair 50S is wide, when manufacturing the diaper 1, the waist elastic members 23 (33) is likely to fit to gaps in the protrusion pattern which forms the welded portion pairs 50S (e.g., protrusions formed on the outer circumferential surface of a roller that holds the sheet). Therefore, when forming the welded portions 50, the waist elastic member 23 (33) can be prevented from being cut off by being crushed and severed by the protrusion pattern, which forms the welded portion pairs 50S.

Further, the welded portion 50 may include a curved portion in a part of its peripheral portion. For example, as shown in FIG. 10B, it may be a quadrilateral with rounded corners, a circular shape, an elliptical shape, a shape with an uneven outline, or the like. In this case as well, the waist elastic member 23 can be slightly inclined with respect to the lateral direction along the curved portion of the welded portion 50, while extending in the lateral direction between the welded portion pairs 50S that are lined up in the lateral direction. This makes it the waist elastic member 23 (33) less likely to come off from the welded portion pair 50S, and the stretchability of the waist portion 2 is maintained.

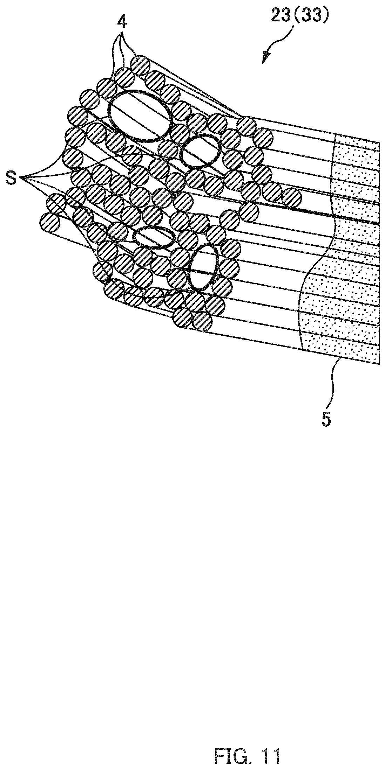

FIG. 11 is an enlarged sectional view of the waist elastic member 23 (33). Examples of the waist elastic member 23 (33) include polyurethane, polyester, and latex (natural rubber). Among these, as shown in FIG. 11, it is preferable to use string-like elastic members in which a plurality of elastic fibers 4 (elastic threads) made from polyurethane, polyester, or the like are aggregated. In this case, the outer shape of the waist elastic member 23 (33) becomes uneven, the surface area where the waist elastic member 23 (33) is in contact with constituent fibers of a sheet increases, and this increases its frictional force with the sheet. This makes it possible to further suppress the coming off of the waist elastic member 23 (33) from the side-joining-portion region 40 and from between the welded portion pair 50S.

In addition, each of the waist elastic members 23 (33) is composed of a plurality of the elastic fibers 4, and this makes the waist elastic member 23 (33) less likely to be cut off when forming the side joining portion 41 by welding or pressurization, making it possible to suppress the coming off of the waist elastic member 23 (33) from the side-joining-portion region 40. More preferably, gaps S are formed between the elastic fibers 4. By doing so, even if the waist elastic member 23 (33) is compressed by the protrusion pattern, which forms the side joining portions 41, the gaps S are compressed, creating an escape margin. This makes the waist elastic member 23 (33) further less likely to cut. Further, when the gap S is compressed, the waist elastic member 23 (33) is more likely to displace in the vertical direction of diaper 1, but it is possible to prevent the waist elastic member 23 (33) from being cut off by being crushed and severed by the protrusion pattern.

Moreover, it is preferable that an oil agent 5 is attached to at least a part of the surface of the waist elastic member 23 (33). By doing so, when forming the side joining portions 41, even if the waist elastic member 23 (33) is stepped on by the protrusion pattern, which forms the side joining portions 41, the waist elastic member 23 (33) is likely to be pushed out of the protrusion pattern because the waist elastic member 23 (22) is slippery due to the oil agent. Therefore, it is possible to suppress the cutting off of the waist elastic member 23 (33).

Further, it is preferable that the weight ratio of the oil agent (the value obtained by dividing the weight of the oil agent by the weight of the waist elastic member 23 (33)) is between 0.5% and 50 (both inclusive). By setting the weight ratio of the oil agent to 0.5% or more, the waist elastic member 23 (33) becomes more slippery, and the waist elastic member 23 (33) becomes more likely to be pushed out of the protrusion pattern, which forms the side joining portions 41. On the other hand, by keeping the weight ratio of the oil agent to 5% or less, it is possible to prevent the frictional force between the sheet and the waist elastic member 23 (33) from excessively deteriorating, making it possible to suppress the coming off of the waist elastic member 23 from the side-joining-portion region 40.

The type of oil agent is not particularly limited, and for example, the oil agent may be one which is applied to the elastic fibers 4, which constitute the waist elastic member 23 (33), for the purpose of preventing sticking, improving workability, etc. Specifically, it is possible to use oil agents mainly composed of: dimethylpolysiloxane; modified polysiloxane in which a portion of the methyl group is substituted with other alkyl groups, phenyl groups, amino groups, etc.; mineral oil; and the like.

Further, the oil agent may be applied not only to the waist elastic member 23 (33) side but also to at least a part of the surface of the sheet facing the waist elastic member 23 (33). In other words, the oil agent may be applied to the non-skin-side surface of the skin-side sheet 21 (31) and the skin-side surface of the non-skin-side sheet 22 (32). In this case as well, the waist elastic member 23 (33) is more likely to slip against the sheet, and is more likely to be pushed out of the protrusion pattern, which forms the side joining portions 41. Therefore, it is possible to suppress the cutting off of the waist elastic member 23 (33).

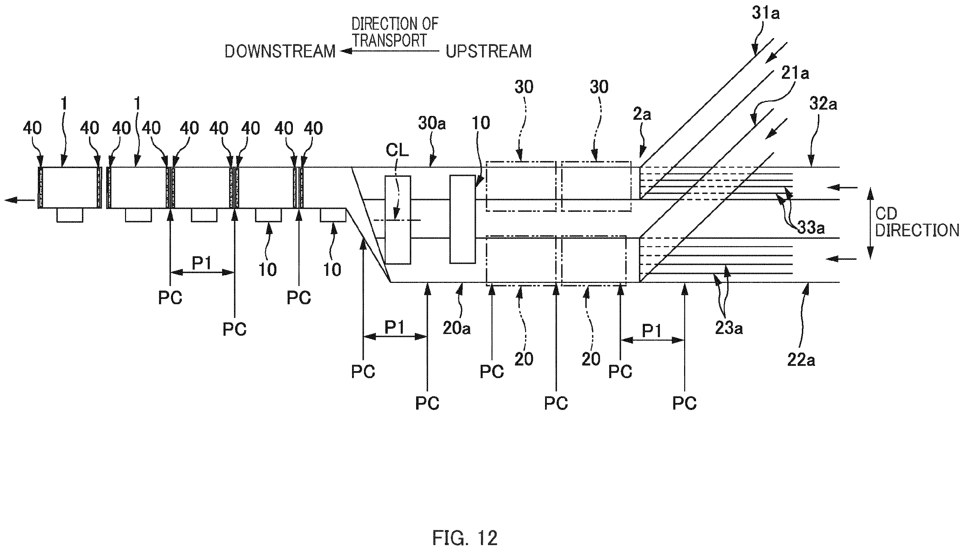

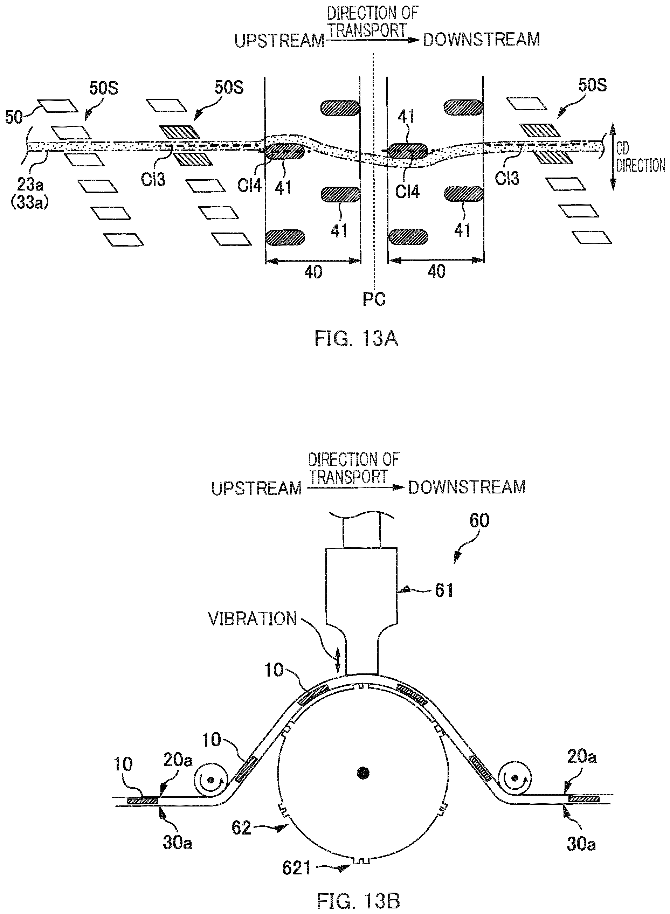

Method for manufacturing underpants-shaped disposable diaper 1 The following describes a method for manufacturing the diaper 1. FIG. 12 is a diagram illustrating the flow of manufacturing the diaper 1 in the manufacturing line. FIG. 13A is a diagram illustrating the formation positions of the side joining portions 41, and FIG. 13B is a schematic side view of an ultrasonic welding device 60 as seen in the CD direction.

In the manufacturing line of the diaper 1 shown in FIG. 12, materials are transported in the direction of transport that corresponds to the lateral direction of the diaper 1. Further, the direction corresponding to the vertical direction of the diaper 1 and intersecting the direction of transport is called the CD direction (intersecting direction). Since the materials for the diaper 1 are continuously transported in the direction of transport, FIG. 12 hypothetically shows the cutting position PC for every product pitch P1.

First, a step of forming the front-waist-portion continuous body 20a and the back-waist-portion continuous body is performed. Specifically, the skin-side-sheet continuous bodies 21a, 31a and a plurality of the waist-elastic-member continuous bodies 23a, 33a that are in a state of stretching in the direction of transport merge with the non-skin-side-sheet continuous bodies 22a, 32a that are being transported in the direction of transport, using transporting devices (not shown) such as transport rollers.

The plurality of the waist-elastic-member continuous bodies 23a, 33a merge in a state where they are lined up side-by-side at intervals in the CD direction. Further, the waist-elastic-member continuous bodies 23a, 33a merge in a state where they stretch to the same value as the maximum stretch factor of the waist elastic members 23 and 33 in the diaper 1. As a result, the waist-elastic-member continuous bodies 23a and 33a are placed between the skin-side-sheet continuous bodies 21a and 31a and the non-skin-side-sheet continuous bodies 22a and 32a, forming a continuous stacked body 2a of the waist portion. Note that the materials to form the front waist portion 20 and the materials to form the back waist portion 30 are transported in a state where they are lined up side-by-side at intervals in the CD direction.

Next, a plurality of the welded portions 50, which joins the skin-side-sheet continuous bodies 21a and 31a and the non-skin-side-sheet continuous bodies 22a and 32a, are formed onto the continuous stacked body 2a of the waist portion being transported to the direction of transport. Although not shown, for example, when forming the welded portions 50 by the ultrasonic welding, it is possible to use an ultrasonic welding device including: an anvil roller that rotates along the direction of transport around the CD direction while wrapping the continuous stacked body 2a of the waist portion; and a horn that vibrates in a direction perpendicular to the outer circumferential surface of the anvil roller. In the outer circumferential surface of the anvil roller, the protrusion pattern (protrusions) corresponding to the welded portions 50 is formed, which receives the vibrations of the horn.

The pattern of the welded portions 50 is one in which the welded portion rows 50L are lined up side-by-side at intervals in the CD direction, and are lined up side-by-side at intervals in the direction of transport, as shown in FIG. 4B described above and the like. Further, the welded portion 50 is formed on both sides of the waist-elastic-member continuous bodies 23a and 33a which are in the stretched state, in the CD direction. In this way, a plurality of the welded portion pairs 50S are formed between which the waist elastic members 23 and 33 of the diaper 1 are sandwiched when the waist elastic members 23 and 33 contract in the lateral direction.

Note that the pattern of the welded portions 50 is preferably formed intermittently for every product pitch P1 so that the welded portion 50 is not formed in the formation positions of the side-joining-portion regions 40, as shown in FIG. 6A and the like. Alternatively, as shown in FIG. 8B, the welded portions 50 may be formed in succession so as to be also located in the formation positions of the side-joining-portion regions 40.

In this way, the front-waist-portion continuous body 20a and the back-waist-portion continuous body 30a are formed. Subsequently, a step of placing the absorbent main bodys 10 is performed. The absorbent main bodys 10 are placed intermittently in the direction of transport every product pitch P1, with the longitudinal direction of the absorbent main body 10 conforming to the CD direction. Further, the absorbent main bodys 10 are fixed spanning across the front-waist-portion continuous body 20a and the back-waist-portion continuous body.

Next, using an unillustrated folding device (guide rollers, guide plates, etc.), each of the absorbent main bodys 10 is folded one time at approximately the center CL in the CD direction, and placed on the front-waist-portion continuous body 20a and the back-waist-portion continuous body, in the thickness direction thereof (the front-back direction).

Then, before forming the side-joining-portion region 40, the waist-elastic-member continuous bodies 23a, 33a in the stretched state is relaxed. For example, the waist-elastic-member continuous body 23a (33a) in the stretched state is relaxed from the state where the waist-elastic-member continuous body 23a (33a) is stretched in the maximum stretch factor of the waist elastic member 23 (33) in the diaper 1, until its length becomes 50% to 70% of its length. However, the front-waist-portion continuous body 20a and the back-waist-portion continuous body 30a are made to be in a state of being stretched appropriately compared to the natural state (no-load state).

This makes it easier to position the materials, making it possible to perform operations at accurate positions, such as to form the side-joining-portion region 40 and to cut each product. Further, the side joining portion 41 can be formed in a state where the basis weight of the sheet (fibers) constituting the f front-waist-portion continuous body 20a and the back-waist-portion continuous body 30a is increased. Therefore, the amount of fibers to be welded (pressurized) increases, and the joining strength of the side joining portions 41 can be increased.

After that, the side-joining-portion region 40 is formed by forming a plurality of the side joining portions 41 arranged side-by-side in the CD direction, the side joining portions 41 being portions that join the front-waist-portion continuous body 20a and the back-waist-portion continuous body that are overlaid in the thickness direction (front-back direction).

As shown in FIG. 13B, when forming the side joining portions 41 by the ultrasonic welding, it is possible to use an ultrasonic welding device 60 including: an anvil roller 62 that rotates along the direction of transport around the CD direction while wrapping materials; and a horn 61 that vibrates in a direction perpendicular to the outer circumferential surface of the anvil roller 62. In the outer circumferential surface of the anvil roller 62, the protrusion pattern (protrusion portions 621) corresponding to the side joining portions 41 is formed, which receives vibrations of the horn 61.

The side-joining-portion regions 40 are formed in pair on both sides of the cutting position PC in the direction of transport, and the pair of the side-joining-portion regions 40 are formed every other product pitch P1. One of the pair of side-joining-portion regions 40 is, for example, the side-joining-portion region 40 of a diaper 1 on the one side in the lateral direction, and the other is the side-joining-portion region 40 of another diaper 1 on the other side in the lateral direction.

Finally, while the stretching of the waist-elastic-member continuous bodies 23a, 33a is relaxed, the f front-waist-portion continuous body 20a and the back-waist-portion continuous body 30a are cut at the cutting position PC every product pitch P1, and an individual diaper 1 is formed.

In the method for manufacturing the diaper 1 according to the present embodiment, in the step of forming the side-joining-portion regions 40, each of the side joining portions 41 is formed so that the center line Cl4 of the side joining portion 41 is shifted with respect to the center line Cl3 of the welded portion pair 50S that is adjacent to the side-joining-portion region 40 in the direction of transport (lateral direction) (e.g., the first welded portion pair 50S1 shown in FIG. 6A and the like), when materials are viewed in the front-back direction (thickness direction) as shown in FIG. 13A.

Note the center line Cl3 of the welded portion pair 50S is a line that bisects with respect to the CD direction between two the welded portions 50 adjacent in the CD direction. The center line Cl4 of the side joining portion 41 is a line that bisects each the side joining portion 41 with respect to the CD direction.

In this way, by shifting the center lines Cl3 and Cl4 in the CD direction, it is possible to reduce the probability that the waist elastic member 23 and 33 that passes between the welded portion pair 50S overlaps the protrusion portion 621 forming the side joining portion 41 (area ratio). Therefore, it is possible to prevent the waist-elastic-member continuous bodies 23a, 33a from being cut off by being crushed and severed by the protrusion portions 621, which form the side joining portions 41. This makes it possible to manufacture the diaper 1 in which the stretchability of the waist portion 2 is maintained.

In addition, in the method for manufacturing the diaper 1 according to the present embodiment, in the step of forming the side-joining-portion regions 40, each of the side joining portions 41 is formed while moving the CD-direction positions of the waist-elastic-member continuous bodies 23a and 33a that are in the state of stretching in the direction of transport. In other words, the protrusion portions 621, which forms the side joining portion 41, are made to push out the waist-elastic-member continuous bodies 23a, 33a without crushing over it. By doing so, it is possible to prevent the waist-elastic-member continuous bodies 23a, 33a from being cut, and to manufacture the diaper 1 in which the stretchability of the waist portion 2 is maintained.

In order to displace the positions of the waist-elastic-member continuous bodies 23a and 33a in the CD direction, for example, the side joining portions 41 are preferably formed by the ultrasonic welding. By doing so, the waist-elastic-member continuous bodies 23a, 33a are made likely to move by the ultrasonic vibration of the horn 61, and are likely to be pushed out of the protrusion portions 621, which form the side joining portions 41. Therefore, it is possible to prevent the waist-elastic-member continuous bodies 23a, 33a from being cut when forming the side joining portion 41.

However, without being limited to the above, the side joining portions 41 may be formed by the welding means different from the ultrasonic welding (e.g., heat welding), or may be formed by non-heating pressurizing means. In addition, in the case of the heat welding, fibers around the side joining portions 41 are also likely to melt due to the influence of heat, but in the case of the ultrasonic welding, mainly fibers of the side joining portion 41 are melt, and the surrounding fibers are less likely to melt. Therefore, it is advisable to confirm the side joining portion 41 has been formed by the ultrasonic welding, by observing the melted state of the fibers around the side joining portion 41.

In addition, as described above, an oil agent may be applied to the surfaces of the waist-elastic-member continuous bodies 23a, 33a, and the surface of the sheet that faces the waist-elastic-member continuous bodies 23a, 33a. In this case as well, the waist-elastic-member continuous bodies 23a, 33a are made likely to move and are more likely to be pushed out of the protrusion portions 621, which form the side joining portions 41 (the position in the CD direction is likely to displace).

Further, it is preferable to cut the waist-elastic-member continuous bodies 23a, 33a into product sizes in a state where their stretching is relaxed. By doing so, compared to the case where the waist-elastic-member continuous bodies 23a, 33a are cut in an stretched state, the amount of the contractive force of the waist elastic members 23, 33 immediately after cutting can be reduced. Therefore, it is possible to suppress the cutting off of the waist elastic members 23 and 33 from the side-joining-portion regions 40 and the welded portion pairs 50S.

### Other Embodiments

The above embodiments of the present disclosure are simply to facilitate understanding of the present disclosure and are not in any way to be construed as limiting the present disclosure. The present disclosure may variously be changed or altered without departing from its gist and encompass equivalents thereof.

### REFERENCE SIGNS LIST

- 1: diaper (underpants-shaped absorbent article),
- 2: waist portion,
- 10: absorbent main body, 11 absorbent core, 12 top sheet,
- 13: back sheet, 14 exterior sheet, 15 leg elastic member,
- 16: leak-proof wall portion,
- 20: front waist portion, 21 skin-side sheet, 22 non-skin-side sheet,
- 23: waist elastic member (elastic member)
- 30: back waist portion, 31 skin-side sheet, 32 non-skin-side sheet,
- 33: waist elastic member (elastic member)
- 40: side-joining-portion region, 41 side joining portion,
- 50: welded portion, 50S welded portion pair,
- 60: ultrasonic welding device, 61 horn, 62 anvil roller,
- 621: protrusion portion

## Claims

1. An underpants-shaped absorbent article having a vertical direction, a lateral direction and a front-back direction that intersect each other,
the underpants-shaped absorbent article comprising:
an absorbent main body; and
a waist portion in which a front waist portion and a back waist portion are joined by a pair of side-joining-portion regions in two ends in the lateral direction,
in the waist portion, a plurality of elastic members being arranged side by side in the vertical direction between a skin-side sheet and a non-skin-side sheet that are joined by a plurality of welded portions,
the plurality of elastic members stretching and contracting in the lateral direction,
the plurality of welded portions being located on both sides of the elastic member in the vertical direction,
a plurality of welded portion pairs being provided in which the elastic member in a state of contracting in the lateral direction is sandwiched and that restricts a position of the elastic member in the lateral direction with respect to the skin-side sheet and the non-skin-side sheet,
the plurality of welded portion pairs having a first welded portion pair and a second welded portion pair,
the first welded portion pair being located adjacent to and laterally inside the side-joining-portion region that is located on a one side in the lateral direction,
the first welded portion pair sandwiching the first elastic member among the plurality of elastic members,
the second welded portion pair being located adjacent to and laterally inside the first welded portion pair,
the second welded portion pair sandwiching the first elastic member,
when the underpants-shaped absorbent article that is in a stretched state is viewed in the front-back direction,
a center line of a portion of the first elastic member that is located in the side-joining-portion region located on a one side in the lateral direction
being inclined with respect to
a center line of a portion of the first elastic member that is located between the first welded portion pair and the second welded portion pair.

2. The underpants-shaped absorbent article according to claim 1, wherein
the first elastic member extends laterally outside the side-joining-portion region that is located on a one side in the lateral direction.

3. The underpants-shaped absorbent article according to claim 1 or 2, wherein
in the side-joining-portion region, a plurality of side joining portions are arranged side by side in the vertical direction, and
a part of the first elastic member is in contact with the side joining portion in the side-joining-portion region on a one side in the lateral direction.

4. The underpants-shaped absorbent article according to claim 1 or 2, wherein
in the side-joining-portion region, a plurality of side joining portions are arranged side by side in the vertical direction, and
in the side-joining-portion region on a one side in the lateral direction, a part of the first elastic member is sandwiched between the skin-side sheet and the non-skin-side sheet by the side joining portion.

5. The underpants-shaped absorbent article according to claim 4, wherein
in the side-joining-portion region on a one side in the lateral direction,
a part of the second elastic member that is among the plurality of the elastic member are in contact with the side joining portion.

6. The underpants-shaped absorbent article according to claim 4, wherein
in the side-joining-portion region of another side in the lateral direction,
a part of the first elastic member is in contact with the side joining portion.

7. The underpants-shaped absorbent article according to any one of claims 1 to 6, wherein
in the side-joining-portion region, a plurality of side joining portions are arranged side by side in the vertical direction, and
a gap in the vertical direction between the side joining portions that are located beside the first welded portion pair in the lateral direction
is shorter than
a gap of the first welded portion pair in the vertical direction.

8. The underpants-shaped absorbent article according to any one of claims 1 to 6, wherein
in the side-joining-portion region, a plurality of side joining portions are arranged side by side in the vertical direction, and
a gap in the vertical direction between the side joining portions that are located beside the first welded portion pair in the lateral direction
is equal to or greater than
a gap of the first welded portion pair in the vertical direction.

9. The underpants-shaped absorbent article according to any one of claims 1 to 8, wherein
in the side-joining-portion region, a plurality of side-joining-portion rows are lined up side-by-side in the lateral direction,
in each of the side-joining-portion rows, a plurality of side joining portions being lined up side-by-side in the vertical direction, and
the plurality of side-joining-portion rows are spaced apart in the lateral direction.

10. The underpants-shaped absorbent article according to any one of claims 1 to 9, wherein
in the side-joining-portion region, a plurality of side-joining-portion rows are lined up side-by-side in the lateral direction,
in each of the side-joining-portion rows, a plurality of side joining portions being lined up side-by-side in the vertical direction,
the side joining portion included in each of the two side-joining-portion rows that are adjacent in the lateral direction is shifted in the vertical direction, and
a length in the vertical direction between the side joining portion that is located adjacent to the first welded portion pair in the lateral direction and the side joining portion that is adjacent the foregoing side joining portion and that is shifted with respect to the foregoing side joining portion in the vertical direction
is smaller than
a diameter of the first elastic member in a state of contracting in the lateral direction.

11. The underpants-shaped absorbent article according to any one of claims 1 to 10, wherein
in the side-joining-portion region, a plurality of side joining portions are arranged side by side in the vertical direction, and
the side-joining-portion region has a portion where the welded portion and the side joining portion overlap in the thickness direction of the waist portion.

12. The underpants-shaped absorbent article according to any one of claims 1 to 11, wherein
in at least either one region of the pair of side-joining-portion regions,
at least one of the elastic members included in the front waist portion is placed shifted in the vertical direction with respect to the elastic member included in the back waist portion.

13. The underpants-shaped absorbent article according to any one of claims 1 to 12, wherein
in the side-joining-portion region, a plurality of side joining portions are arranged side by side in the vertical direction, and
letting a times be the stretch factor of the first elastic member when the underpants-shaped absorbent article is stretched to a maximum extent in the lateral direction,
letting D mm be a width of the first elastic member in the vertical direction when the underpants-shaped absorbent article is stretched to a maximum extent in the lateral direction,
letting *b* times a maximum stretch factor of the first elastic member alone,
a vertical length of the side joining portion that is located beside the first welded portion pair in the lateral direction is equal to or less than D × *b*/*a* mm.

14. The underpants-shaped absorbent article according to any one of claims 1 to 13, wherein
a distance in the vertical direction between the two welded portions that constitute the welded portion pair is wider in at least an end portion located on a one side in the lateral direction than in a central portion of the welded portion pair in the lateral direction.

15. The underpants-shaped absorbent article according to any one of claims 1 to 14, wherein
an oil agent is attached to at least a portion of a surface of the elastic member.

16. The underpants-shaped absorbent article according to any one of claims 1 to 15, wherein
the elastic member is a string-like elastic member in which a plurality of elastic fibers are aggregated.

17. A method for manufacturing an underpants-shaped absorbent article,
the underpants-shaped absorbent article having a vertical direction, a lateral direction and a front-back direction that intersect each other,
the underpants-shaped absorbent article including
an absorbent main body and
a waist portion in which a front waist portion and a back waist portion are joined by a pair of side-joining-portion regions in two ends in the lateral direction,
the method comprising:
a step of forming a continuous body of the front waist portion and a continuous body of the back waist portion, by forming a plurality of welded portion pairs when forming a plurality of welded portions after placing a plurality of continuous bodies of an elastic member between a skin-side-sheet continuous body and a non-skin-side-sheet continuous body,
the plurality of continuous bodies of the elastic member being in a state of stretching in a direction of transport corresponding to the lateral direction,
the plurality of continuous bodies of the elastic member being placed side-by-side in an intersecting direction of the direction of transport,
the plurality of welded portions being portions that join the skin-side-sheet continuous body and the non-skin-side-sheet continuous body,
the skin-side-sheet continuous body and the non-skin-side-sheet continuous body being continuous in the direction of transport,
the plurality of welded portion pairs holding composed of the welded portions that are placed on both sides of each of the plurality of continuous bodies of the elastic member in the intersecting direction,
when the elastic member in the underpants-shaped absorbent article contracts in the lateral direction, the plurality of welded portion pairs holding the elastic member and restricting a position of the elastic member in the lateral direction with respect to the skin-side sheet and the non-skin-side sheet; and
a step of forming a side-joining-portion region by forming a plurality of side joining portions arranged side-by-side in the intersecting direction,
the plurality of side joining portions being portions for joining the continuous body of the front waist portion and the continuous body of the back waist portion that are overlaid in a thickness direction,
in the step of forming the side-joining-portion region,
the side joining portion being formed so that a center line of the side joining portion is shifted with respect to a center line of the welded portion pair that is adjacent to the side-joining-portion region in the direction of transport, when materials are viewed in the front-back direction.

18. A method for manufacturing an underpants-shaped absorbent article,
the underpants-shaped absorbent article having a vertical direction, a lateral direction and a front-back direction that intersect each other,
the underpants-shaped absorbent article including
an absorbent main body and
a waist portion in which a front waist portion and a back waist portion are joined by a pair of side-joining-portion regions in two ends in the lateral direction,
the method comprising:
a step of forming a continuous body of the front waist portion and a continuous body of the back waist portion, by forming a plurality of welded portion pairs when forming a plurality of welded portions after placing a plurality of continuous bodies of an elastic member between a skin-side-sheet continuous body and a non-skin-side-sheet continuous body,
the plurality of continuous bodies of the elastic member being in a state of stretching in a direction of transport corresponding to the lateral direction,
the plurality of continuous bodies of the elastic member being placed side-by-side in an intersecting direction of the direction of transport,
the plurality of welded portions being portions that join the skin-side-sheet continuous body and the non-skin-side-sheet continuous body,
the skin-side-sheet continuous body and the non-skin-side-sheet continuous body being continuous in the direction of transport,
the plurality of welded portion pairs holding composed of the welded portions that are placed on both sides of each of the plurality of continuous bodies of the elastic member in the intersecting direction,
when the elastic member in the underpants-shaped absorbent article contracts in the lateral direction, the plurality of welded portion pairs holding the elastic member and restricting a position of the elastic member in the lateral direction with respect to the skin-side sheet and the non-skin-side sheet; and
a step of forming a side-joining-portion region by forming a plurality of side joining portions arranged side-by-side in the intersecting direction,
the plurality of side joining portions being portions for joining the continuous body of the front waist portion and the continuous body of the back waist portion that are overlaid in a thickness direction,
in the step of forming the side-joining-portion region,
the side joining portion being formed while moving an intersecting-direction position of the continuous bodies of the elastic member that are in the state of stretching in the direction of transport.

19. The method for manufacturing an underpants-shaped absorbent article according to claim 17 or 18, wherein
in the step of forming the side-joining-portion region,
the side joining portion is formed by the ultrasonic welding.
